# EUROPEAN PATENT SPECIFICATION

(11) **EP 1 482 971 B1**
(45) Date of publication and mention of the grant of the patent: **09.10.2013**
(21) Application number: 03742468.6
(22) Date of filing: 18.02.2003
(51) Int. Cl.: A61K 39/00, A61P 31/18, G01N 33/569

(54) **STRATEGY FOR RETROVIRAL IMMUNOTHERAPY**
STRATEGIE FÜR DIE RETROVIRAL IMMUNTHERAPIE
STRATEGIE D'IMMUNOTHERAPIE RETROVIRALE

(30) Priority: 20.02.2002 AU PS065002
(43) Date of publication of application: 08.12.2004
(73) Proprietor: Immunaid Pty Ltd, Fitzroy, Victoria 3065 (AU)
(72) Inventor: ASHDOWN, Martin, Leonard, Thornbury, Victoria 3071 (AU)
(74) Representative: Harding, Charles Thomas
(86) International application number: PCT/AU2003/000207
(87) International publication number: WO 2003/070270

(56) References cited:
- EP-B- 0 374 207
- WO-A-02/13828
- WO-A1-2005/040816
- ERRANTE D ET AL: "HODGKIN'S DISEASE IN 35 PATIENTS WITH HIV INFECTION:AN EXPERIENCE WITH EPIRUBICIN, BLEOMYCIN, VINBLASTINE AND PREDNISONE CHEMOTHERAPY IN COMBINATION WITH ANTIRETROVIRAL THERAPY AND PRIMARY USE OF G-SCF" MEDLINE, February 1999 (1999-02), XP002964471
- LORI F: "Hydroxyurea and HIV: 5 years later--from antiviral to immune-modulating effects." AIDS (LONDON, ENGLAND) 20 AUG 1999, vol. 13, no. 12, 20 August 1999 (1999-08-20), pages 1433-1442, XP002488508 ISSN: 0269-9370
- BEILHARZ MANFRED W ET AL: "Prevention of murine AIDS by timed immune regulator cell ablation" JOURNAL OF INTERFERON AND CYTOKINE RESEARCH, MARY ANN LIEBERT, NEW YORK, NY, US, vol. 22, no. Supplement 1, October 2002 (2002-10), pages S-177, XP008094178 ISSN: 1079-9907
- DAAR E S ET AL: "Acute HIV syndrome after discontinuation of antiretroviral therapy in a patient treated before seroconversion." ANNALS OF INTERNAL MEDICINE 15 MAY 1998, vol. 128, no. 10, 15 May 1998 (1998-05-15), pages 827-829, XP002488509 ISSN: 0003-4819
- MELCHIOR J C ET AL: "Malnutrition and wasting, immunodepression, and chronic inflammation as independent predictors of survival in HIV-infected patients." NUTRITION (BURBANK, LOS ANGELES COUNTY, CALIF.) 1999 NOV-DEC, vol. 15, no. 11-12, November 1999 (1999-11), pages 865-869, XP002488510 ISSN: 0899-9007
- MANFRED W BEILHARZ ET AL: "Timed Ablation of Regulatory CD4 T Cells Can Prevent Murine AIDS Progression" JOURNAL OF IMMUNOLOGY, THE WILLIAMS AND WILKINS CO. BALTIMORE, vol. 172, no. 8, 15 April 2004 (2004-04-15), pages 4917-4925, XP007905048 ISSN: 0022-1767
- IWASHIRO M ET AL: "Immunosuppression by CD4+ regulatory T cells induced by chronic retroviral infection." PROCEEDINGS OF THE NATIONAL ACADEMY OF SCIENCES OF THE UNITED STATES OF AMERICA 31 JUL 2001, vol. 98, no. 16, 31 July 2001 (2001-07-31), pages 9226-9230, XP002488511 ISSN: 0027-8424
- NORTH R J ET AL: "Elimination of cycling CD4+ suppressor T cells with an anti-mitotic drug releases non-cycling CD8+ T cells to cause regression of an advanced lymphoma." IMMUNOLOGY SEP 1990, vol. 71, no. 1, September 1990 (1990-09), pages 90-95, XP002488512 ISSN: 0019-2805
- IMAMI N ET AL: "Development of immunotherapeutic strategies for HIV-1." EXPERT OPINION ON BIOLOGICAL THERAPY SEP 2001, vol. 1, no. 5, September 2001 (2001-09), pages 803-816, XP008094213 ISSN: 1471-2598
- MORTENSEN R F: "C-reactive protein, inflammation, and innate immunity." IMMUNOLOGIC RESEARCH 2001, vol. 24, no. 2, 2001, pages 163-176, XP002488513 ISSN: 0257-277X
- MORSE H C 3RD ET AL: "Retrovirus-induced immunodeficiency in the mouse: MAIDS as a model for AIDS." AIDS (LONDON, ENGLAND) JUL 1992, vol. 6, no. 7, July 1992 (1992-07), pages 607-621, XP008094218 ISSN: 0269-9370
- SALOMON B. ET AL.: 'B7/CD28 costimulation is essential for the homeostasis of the CD4+CD25+ immunoregulatory T cells that control autoimmune diabetes' IMMUNITY vol. 12, 2000, pages 431 - 440, XP008040741
- SURI-PAYER E. ET AL.: 'Differential cytokine requirements for regulation of autoimmune gastritis and colitis by CD4+CD25+ T cells' JOURNAL OF AUTOIMMUNITY vol. 16, 2001, pages 115 - 123, XP008040740
- SHIMIZU J. ET AL.: 'Stimulation of CD25+CD4+ regulatory T cells through GITR breaks immunological self-tolerance' NATURE IMMUNOLOGY vol. 3, no. 2, January 2002, pages 135 - 142, XP002247979
- SAKAGUCHI, S.: "Regulatory TCells: Key controllers of Immunologic Self-Tolerance", CELL, vol. 101, 28 May 2000 (2000-05-28), pages 455-458,
- READ, S. AND POWRIE, F.: "CD4+ regulatory T cells", CURRENT OPINION IN IMMUNOLOGY, vol. 13, 2001, pages 644-649,
- KNOECHEL ET AL.: "Sequential development of interleukin 2-dependent effector and regulatory T cells in response to endogenous systemic antigen.", JEM, vol. 202, 21 November 2005 (2005-11-21), pages 1-12,
- HIURA, T. ET AL: "Both Regulatory T Cells and Antitumor Effector T Cells are Primed in the Same Draining Lymph Nodes during Tumor Progression", THE JOURNAL OF IMMUNOLOGY, 2005, pages 5058-5066,
- DARRASSE-JÈZE, G. ET AL.: 'Tumor emergence is sensed by self-specific CD44hi memory Tregs that create a dominant tolerogenic environment for tumors in mice.' THE JOURNAL OF CLINICAL INVESTIGATION pages 1 - 15
- VAHLENKAMP, T.W. ET AL.: "Feline Immunodeficiency Virus Infection Phenotypically and Functionally Activates Immunosuppressive CD4+CD25+ T Regulatory Cells", THE JOURNAL OF IMMUNOLOGY, vol. 172, 2004, pages 4752-4761,
- HRYNIEWICZ, A. ET AL.: "CTLA-4 blockade decreases TGF-beta, IDO, and viral RNA expression in tissues of SIVmac251-infected macaques", IMMUNOBIOLOGY, vol. 108, no. 2, 8 August 2006 (2006-08-08), pages 3834-3842,
- ESTES, J.D. ET AL.: "Simian immunodeficiency virus-induced lymphatic tissue fibrosis is mediated by transforming growth factor beta 1-positive regulatory T cells and begins in early infection.", THE JOURNAL OF INFECTIOUS DISEASES, vol. 195, 15 February 2007 (2007-02-15), pages 551-561,
- WEISS, L. ET AL.: "Human immunodeficiency virus-driven expansion of CD4+CD25+ regulatory T cells, which suppress HIV-specific CD4 T-cell responses in HIV-infected patients", BLOOD, vol. 104, 15 November 2004 (2004-11-15), pages 3249-3256,
- KINTER, A.L. ET AL.: "CD25(+)CD4(+) regulatory T cells from the peripheral blood of asymptomatic HIV-infected individuals regulate CD4(+) and CD8(+) HIV-specific T cell immune responses in vitro and are associated with favorable clinical markers of disease status.", THE JOURNAL OF EXPERIMENTAL MEDICINE, vol. 200, no. 3, 2 August 2004 (2004-08-02), pages 331-343,
- LIM, A.Y-F. ET AL.: "Cell surface markers of regulatory T cells are not associated with increased forkhead box p3 expression in blood CD4+ T cells from HIV-infected patients responding to antiretroviral therapy", IMMUNOLOGY AND CELL BIOLOGY, 2006, pages 1-7,
- NILSSON, J. ET AL.: "HIV-1-driven regulatory T-cell accumulation in lymphoid tissues is associated with disease progression in HIV/AIDS", IMMUNOBIOLOGY, vol. 108, 1 December 2008 (2008-12-01), pages 3808-3817,
- KINTER AUDREY ET AL: "Suppression of HIV-specific T cell activity by lymph node CD25+ regulatory T cells from HIV-infected individuals.", PROCEEDINGS OF THE NATIONAL ACADEMY OF SCIENCES OF THE UNITED STATES OF AMERICA 27 FEB 2007, vol. 104, no. 9, 27 February 2007 (2007-02-27), pages 3390-3395, ISSN: 0027-8424
- KINTER AUDREY L ET AL: "CD25+ regulatory T cells isolated from HIV-infected individuals suppress the cytolytic and nonlytic antiviral activity of HIV-specific CD8+ T cells in vitro.", AIDS RESEARCH AND HUMAN RETROVIRUSES MAR 2007, vol. 23, no. 3, March 2007 (2007-03), pages 438-450, ISSN: 0889-2229
- LIM ANDREW ET AL: "Proportions of circulating T cells with a regulatory cell phenotype increase with HIV-associated immune activation and remain high on antiretroviral therapy.", AIDS (LONDON, ENGLAND) 31 JUL 2007, vol. 21, no. 12, 31 July 2007 (2007-07-31) , pages 1525-1534, ISSN: 0269-9370
- CAO WEIWEI ET AL: "Regulatory T cell expansion and immune activation during untreated HIV type 1 infection are associated with disease progression.", AIDS RESEARCH AND HUMAN RETROVIRUSES FEB 2009, vol. 25, no. 2, February 2009 (2009-02), pages 183-191, ISSN: 1931-8405
- VAHLENKAMP, T.W. ET AL.: "The role of CD24+CD25+ regulatory T cells in viral infections", VETERINARY IMMUNOLOGY AND IMMUNOPATHOLOGY, vol. 108, 2005, pages 219-225,
- BELKAID YASMINE ET AL: "Natural regulatory T cells in infectious disease.", NATURE IMMUNOLOGY APR 2005, vol. 6, no. 4, April 2005 (2005-04), pages 353-360, ISSN: 1529-2908
- ROUSE, B.T. ET AL: "Regulatory T cells in virus infection", IMMUNOLOGICAL REVIEWS, vol. 212, 2006, pages 272-286,
- DITTMER ULF ET AL: "Functional impairment of CD8(+) T cells by regulatory T cells during persistent retroviral infection.", IMMUNITY MAR 2004, vol. 20, no. 3, March 2004 (2004-03), pages 293-303, ISSN: 1074-7613
- "13" In: Janis Kuby: "Immunology", 1994 pages 313-315, 605,
- EDITH ROUMEN KLAPPE ET AL: 'Inflammatory response in the acute phase of deep vein thrombosis' JOURNAL OF VASCULAR SURGERY vol. 35, no. 4, 01 April 2002, pages 701 - 706, XP055000781 DOI: 10.1067/mva.2002.121746 ISSN: 0741-5214
- GABAY C ET AL: "Acute-phase proteins and other systemic responses to inflammation.", THE NEW ENGLAND JOURNAL OF MEDICINE 11 FEB 1999 LNKD- PUBMED:9971870, vol. 340, no. 6, 11 February 1999 (1999-02-11), pages 448-454, ISSN: 0028-4793

## Description

### FIELD OF THE INVENTION:

Disclosed herein is a method of treating a retroviral infection in a mammalian subject. Disclosed herein is a method of treating a retroviral infection which leads to an immunodeficiency-related disease in a human subject.

### BACKGROUND OF THE INVENTION:

Human immunodeficiency virus (HIV) induces a persistent and progressive infection leading, in the vast majority of cases, to the development of the acquired immunodeficiency syndrome (AIDS). There are at least two distinct types of HIV: HIV-1 and HIV-2. In humans, HIV infection eventually leads to immune incompetence, opportunistic infections, neurological dysfunctions, neoplastic growth, and ultimately death.

HIV is a member of the lentivirus family of retroviruses. Retroviruses are small enveloped viruses that contain a single-stranded RNA genome, and replicate via the insertion of a DNA intermediate into the host DNA produced by a virally-encoded reverse transcriptase. Other retroviruses include, for example, oncogenic viruses such as human T-cell leukemia viruses (HTLV-I,-II,-III), feline leukemia virus, and the murine type C retroviruses.

The HIV viral particle consists of a viral core, composed in part of capsid proteins designated p24 and p18, together with the viral RNA genome and those enzymes required for early replicative events. Myristylated gag protein forms an outer viral shell around the viral core, which is, in turn, surrounded by a lipid membrane envelope derived from the infected cell membrane. The HIV envelope surface glycoproteins are synthesized as a single 160 kilodalton precursor protein which is cleaved by a cellular protease during viral budding into two glycoproteins, gp41 and gp120. gp41 is a transmembrane glycoprotein and gp120 is an extracellular glycoprotein which remains non-covalently associated with gp41, possibly in a trimeric or multimeric form.

HIV infection is pandemic and HIV-associated diseases represent a major world health problem. Although considerable effort is being put into the design of effective therapeutics, currently no curative anti-retroviral drugs or therapies against AIDS exist. In attempts to develop such drugs, several stages of the HIV life cycle have been considered as targets for therapeutic intervention (Mitsuya et al., 1991).

Attention has been given to the development of vaccines for the treatment of HIV infection. The HIV-1 envelope proteins (gp160, gyp120, gp41) have been shown to be the major antigens for anti-HIV antibodies present in AIDS patients (Barin et al., 1985). Thus far, therefore, these proteins seem to be the most promising candidates to act as antigens for anti-HIV vaccine development. Several groups have begun to use various portions of gp160, gp120, and/or gp41 as immunogenic targets for the host immune system (US 5,141,867; WO 92/22654; WO 91/09872; WO 90/07119; US 6,090,392). Despite these efforts, an effective vaccine strategy for the treatment of HIV infection has not been developed.

Disclosed herein is an alternate immunotherapy for treating a retroviral infection.

EP0374207 relates to a drug containing a component which targets regulator T cells and a target which activates effector T cells. Errante et al. (Ann Oncol 1999, 10:189-95) relates to methods of treating HIV patients with cancer, whereby antiretroviral therapy is provided to treat the HIV infection and vinblastine administered to treat the cancer. Lori (AIDS 1999,13(12):1433-1442) provides a review of the use of hydroxyurea to treat HIV. WO 02/13828 relates to methods of resetting the immune system to treat a retroviral infection.

### SUMMARY OF THE INVENTION:

The present invention provides methods and uses as defined in the appended claims.

The present inventor has noted that at least two populations of immune cells are produced in response to retroviruses which infect mammals. More particularly, the immune system of a mammal infected with a retrovirus is capable of mounting an immune response against the virus through a group of cells herein generally referred to as "effector cells", however, a second population of cells are also produced which regulate the "effector cells", herein generally referred to as "regulator cells", limiting the mammal's ability to effectively control or eradicate the retroviral infection.

Whilst not wishing to be limited by theory, it is proposed that humans contain many sequences which are homologous or near homologous to retroviral sequences fragmented throughout their genome as stable heritable elements. Accordingly, proteins encoded by these sequences may be recognised as "self" during development of the immune response. Subsequent infection by a retrovirus may then also be partially recognized as "self", limiting the immune system's ability to mount a successful immune response. This proposal, at least in part, may explain why up until now it has been difficult to develop an effective vaccine against HIV.

Support for this hypothesis has been provided by Rakowicz-Szulczynska and co-workers (1998 and 2000) who have shown that some antigens associated with breast cancer are molecularly and immunologically similar to proteins encoded by HIV-1. Similar observations have been made for other cancers. In addition, Coll et al. (1995) have reported antibodies which bind HIV-1 in patients with autoimmune diseases such as Sjogren's syndrome and systemic lupus erythematosus. Furthermore, a BLAST search of the human genome database with the human immunodeficiency virus genome sequences indicates many regions of significant identity between the two genomes.

It has also been noted that the relative number of effector cells expand in response to an antigen before the regulator cells expand. This provides an opportunity to prevent the production of, limits the function of, or destroy, the "regulator cells" whilst maintaining the "effector cells".

Furthermore, the present inventor has also found that levels of acute phase inflammatory markers can be used as an indicator of when an agent directed against the "regulator cells" can be administered in the treatment of a retroviral infection.

Disclosed herein is a method of treating a retroviral infection in a mammalian subject, the method comprising administering to the subject a composition which increases the number of, and/or activates, effector cells directed against the retrovirus, and subsequently administering to the subject an agent which inhibits the production of, limits the function of, and/or destroys, regulator cells, wherein the timing of administration of the agent is selected such that the activity of the effector cells is not significantly reduced, and wherein fluctuations in the levels of an acute phase inflammatory marker in the subject is used to assist in determining when the agent is administered.

There are many ways in which the number, and/or activity, of effector cells directed against a retrovirus can be increased. In some instances, this will occur by an inadvertent infection with the retrovirus, for example, a needle prick of a syringe containing the virus. As is well known, health workers and researchers run the risk of viral infection through needle stick injury. Similarly, the general public are also exposed to this danger through discarded syringes, particularly on the beach, and muggings with such syringes. Therefore, upon suspected exposure to a retrovirus, particularly HIV, the mammalian subject could utilize the method of the present invention.

The method can also be used to treat a mammalian subject which has been infected with a retrovirus for some time. Although the immune system of such a subject has already been exposed to the retrovirus, the addition of further retroviral antigens could lead to a further effector cell response with subsequent opportunity to ablate the regulators of these new effectors.

Subjects infected with a retrovirus can be treated with antiretroviral drugs, such as in HAART treatment, to keep the viral load low. Such subjects also could be administered with the composition to initiate a new effector cell immune response whilst the subsequent administration of the agent would ablate regulator cells.

Fluctuations in the levels of the acute phase inflammatory marker is monitored to determine when the agent is administered.

As known in the art, some acute phase inflammatory markers initially increase during an immune response (referred to hereinafter as positive acute phase inflammatory markers) whilst others initially decrease during an immune response (referred to hereinafter as negative acute phase inflammatory markers).

Preferably, the acute phase inflammatory marker is a positive acute phase inflammatory marker, and the agent is administered approximately when the levels of the positive acute phase inflammatory marker begin to decrease after an initial increase in levels of the marker. In this instance, effector cell production and/or activity is indicated by increased levels of the positive acute phase inflammatory marker. Upon clonal expansion of regulator cells, the activity of effector cells is downregulated resulting in a decrease in positive acute phase inflammatory marker levels.

Preferably, the positive acute phase inflammatory marker is c-reactive protein.

In a particularly preferred embodiment, the agent is administered approximately when the levels of c-reactive protein have peaked and begun to decrease.

It has also been discovered that acute phase inflammatory markers can be an indicator of viral load because an effective immune response results in lower viral load which in turn leads to lower acute phase inflammatory marker levels. Since acute phase inflammatory marker levels can be readily measured in small blood samples it can be used as an indicator of when to start monitoring viral load.

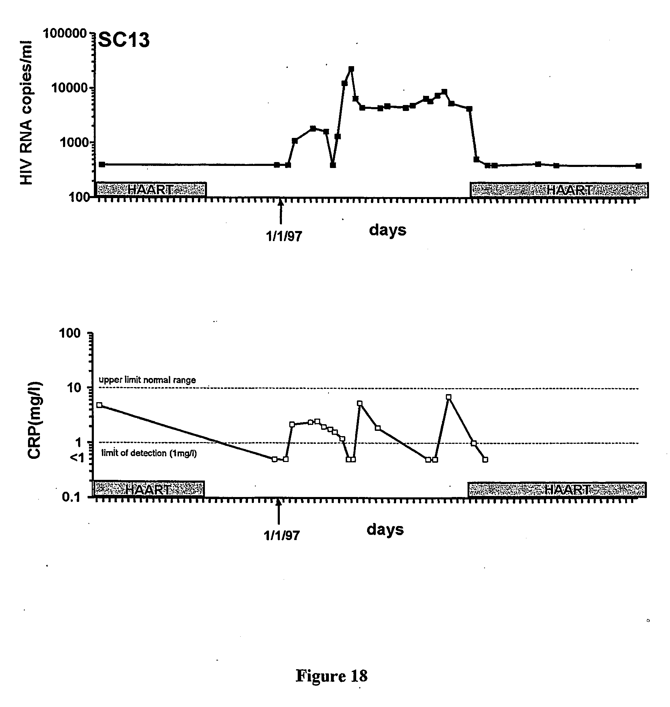

Thus, in another preferred embodiment, the agent is administered approximately when the number of viral particles has begun to stabilize or increase following administration of the composition, wherein testing for viral particle levels in the subject begins when the positive acute phase inflammatory marker levels begins to increase following administration of the composition. It should be noted that the timing of administering the agent can also coincide with a decrease in the number of viral particles, after an initial peak (as seen in Figure 18).

Preferably, the positive acute phase inflammatory marker is c-reactive protein.

In yet another preferred embodiment of the first aspect, restimulation of effector cells in an infected mammalian subject can be achieved by a composition which comprises a retroviral antigenic polypeptide. Preferably, the antigenic polypeptide is provided to the subject by administering a vaccine comprising the retrovirus antigenic polypeptide and a pharmaceutically acceptable carrier. More preferably, the vaccine further comprises an adjuvant.

In another embodiment the antigenic polypeptide is provided to the subject by administering a DNA vaccine encoding the retroviral antigenic polypeptide.

In yet another embodiment, the antigenic polypeptide is provided to the subject by the consumption of a transgenic plant expressing the retroviral antigenic polypeptide.

Withdrawal of antiretroviral treatment typically causes a rapid re-expansion of effector cells against the re-emergent virus in an infected subject. Accordingly, at the appropriate time the agent can be administered to ablate the regulator cells without the need to administer a composition as defined herein.

Disclosed herein is a method of treating a retroviral infection in a mammalian subject, the method comprising exposing the subject to antiretroviral drug therapy, and subsequently administering to the subject an agent which inhibits the production of, limits the function of, and/or destroys, regulator cells, wherein the agent is administered after the antiretroviral drug therapy has concluded and a resulting expansion in retroviral numbers has led to an increase in the number, and/or activation, of effector cells directed against the retrovirus, and wherein the timing of administration of the agent is selected such that the activity of effector cells is not significantly reduced, and wherein fluctuations in the levels of an acute phase inflammatory marker are used to assist in determining when the agent is administered.

Preferably, the acute phase inflammatory marker is a positive acute phase inflammatory marker. Preferably, the acute phase inflammatory marker is c-reactive protein.

In a preferred embodiment, the agent is administered approximately when the levels of c-reactive protein have peaked and begun to decrease.

Preferably, the antiretroviral drug therapy is HAART.

Upon removal of antiretroviral drug therapy the viral load increases (for example, see Daar et al., 1998 and Figure 18). This results in an expansion and/or activation of effector cells directed against the retrovirus which begin to stabilize the viral load. It is approximately when the retroviral numbers have peaked, or begun to decrease after this peak, that the agent should be administered. As outlined above, acute phase inflammatory marker levels can be an indicator of viral load.

Accordingly, in a further preferred embodiment of the second aspect, the agent is administered approximately when the number of viral particles has peaked, or begun to decrease after this peak, in response to the conclusion of the antiretroviral drug therapy, wherein testing for viral particle levels in the subject begins when the positive acute phase inflammatory marker levels begin to increase following conclusion of the antiretroviral drug therapy.

Disclosed herein is the use of a composition which increases the number of, and/or activates, effector cells directed against a retrovirus for the manufacture of a medicament for administering to a mammalian subject with a retroviral infection, wherein the subject is subsequently administered with an agent which inhibits the production of, limits the function of, and/or destroys, regulator cells, and wherein the timing of administration of the agent is selected such that the activity of the effector cells is not significantly reduced, and wherein fluctuations in the levels of an acute phase inflammatory marker in the subject is used to assist in determining when the agent is administered.

Disclosed herein is the use of an agent which inhibits the production of, limits the function of, and/or destroys, regulator cells for the manufacture of a medicament for administering to a mammalian subject with a retroviral infection, wherein the subject has previously been administered with a composition which increases the number of, and/or activates, effector cells directed against the retrovirus, and wherein the agent is administered at a time selected such that the activity of the effector cells is not significantly reduced, and wherein fluctuations in the levels of an acute phase inflammatory marker in the subject is used to assist in determining when the agent is administered.

Disclosed herein is the use of an antiretroviral drug for the manufacture of a medicament for administering to a mammalian subject with a retroviral infection, wherein the subject is subsequently administered with an agent which inhibits the production of, limits the function of, and/or destroys, regulator cells, and wherein the timing of administration of the agent is selected such that the activity of effector cells directed against the retrovirus is not significantly reduced, and wherein fluctuations in the levels of an acute phase inflammatory marker in the subject is used to assist in determining when the agent is administered.

Disclosed herein is the use of an agent which inhibits the production of, limits the function of, and/or destroys, regulator cells for the manufacture of a medicament for administering to a mammalian subject with a retroviral infection, wherein the subject has previously been administered with an antiretroviral drug, and wherein the agent is administered at a time selected such that the activity of effector cells directed against the retrovirus is not significantly reduced, and wherein fluctuations in the levels of an acute phase inflammatory marker in the subject is used to assist in determining when the agent is administered.

Preferably, the agent used in regulator cell ablation is selected from the group consisting of anti-proliferative drugs, radiation, and antibodies which inhibit the down regulation activity of the regulator cells. Preferably, the anti-proliferative drug is selected from the group consisting of vinblastine and anhydro vinblastine.

Examples of preferred antibodies include, but are not limited to, anti-CD4+, anti-CTLA-4 (cytotoxic lymphocyte-associated antigen-4), and anti-CD28.

Preferably, the retrovirus is selected from the group consisting of HIV-1, HIV-2, HTLV-1 and HTLV-2.

As would be readily appreciated by those skilled in the art, the methods of the present invention may be repeated to provide a more complete treatment.

Preferably, the mammalian subject is a human.

Disclosed herein is a kit comprising;
i) a composition for increasing the number of, and/or activating, effector cells directed against a retrovirus in a mammalian subject;
ii) an agent which inhibits the production of, limits the function of, and/or destroys, regulator cells; and
iii) means for determining the level of an acute phase inflammatory marker.

In another aspect, the present invention provides a kit comprising;
i) at least one antiretroviral drug;
ii) an agent which inhibits the production of, limits the function of, and/or destroys, regulator cells; and
iii) means for determining the level of an acute phase inflammatory marker.

In each aspect outlined above, ablation of the "regulator" population allows the "effector" population to reduce or eradicate the retroviral load as any down regulation of effectors (due to self-tolerance mechanisms) has been removed.

As will be apparent, preferred features and characteristics of one aspect of the disclosure can be applicable to other aspects of the disclosure.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention will hereinafter be described by way of the following non-limiting Figures and Examples.

### BRIEF DESCRIPTION OF THE DRAWINGS:

Figure 1: Time course of MAIDS in B6 mice infected with LP-BM5.
Figure 2: Effect of a single dose of vinblastine (6mg/kg i.p.) on MAIDS progression at 10 weeks post infection.
Figure 3: Spleen histology of vinblastine treated mice 10 weeks post infection.
Figure 4: Protection from MAIDS at 20 weeks post infection following vinblastine therapy. n=5.
Figure 5: Rechallenge with MAIDS virus following protective vinblastine therapy n=5.
Figure 6: Effect of day 14 vinblastine on spleen cell percentages in MAIDS infected (MAIDS+) and control mice (MAIDS-). n=3.
Figure 7: Spleen transfer experimental protocol.
Figure 8: Spleen cell transfers from MAIDS infected donor mice. n=7.
Figure 9: *In vivo* depletion experimental protocol.
Figure 10: *In vivo* depletion of CD4+ or CD8+ cells at day 14 post infection n=5.
Figure 11: Flowchart of the protocol for the adoptive transfer experiment described in Section 4 of the Examples.
Figure 12: Results of CD4+CD25+/- adoptive transfers using uninfected donor cells.
Spleen weights were taken at 10 weeks post MAIDS infection (n=5 mice per group).
Figure 13: IL-4 in serum of MAIDS infected mice. Each time point represents the mean+/-SEM for 9 mice.
Figure 14: IL-10 in serum of MAIDS infected mice. Each time point represents the mean+/-SEM for 9 mice.
Figure 15: IL-4 and IL-10 levels as determined by intracellular flow in total WBC from spleen. Data shown is the mean+/-SEM for 3 mice per group.
Figure 16: IL-4 production by ELISpot of splenic WBCs of MAIDS infected mice.
Data shown is the mean+/-SEM for 3 mice per group.
Figure 17: IL-10 production by ELISpot of splenic WBCs of MAIDS infected mice.
Data shown is the mean+/-SEM for 3 mice per group.
Figure 18: HIV RNA, c-reactive protein and T cell levels in response to taking a human patient offHAART treatment.

### DETAILED DESCRIPTION OF THE INVENTION:

### Definitions

As used herein the term "treating" or "treat" means a reduction in retroviral load is achieved. Most preferably, the retroviral load is completely eradicated.

"Regulator cells" include, but are not necessarily limited to, a subpopulation of CD4+ T cells. Such cells may also be referred to in the art as "suppressor cells". Regulator cells may either act directly on effector cells or may assert their affects upon effector cells through other mechanisms.

CD4+ cells express the marker known in the art as CD4. Typically, the term "CD4+ T cells" as used herein does not refer to cells which also express CD8. However, this term can include T cells which also express other antigenic markers such as CD25.

"Effector cells" include, the T cell population known as CD8+ cells.

As used herein, the term "ablate" or "ablation" when referring to the exposure of the "regulator cells" to the agent means that the number, and/or activity, of regulator cells is down-regulated by the agent. Most preferably, the number, and/or activity, of regulator cells is completely eradicated by the agent.

Unless otherwise indicated, the recombinant DNA and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

### Agents which Inhibit the Production of, Limits the Function of, and/or Destroy, Regulator Cells

The agent selectively or non-selectively results in the destruction, or the inhibition of the production, of regulator cells. For example, a CD4+ specific antibody could be used to specifically target CD4+ T cells. However, in some instances a non-selective agent could be used, such as an anti-proliferative drug or radiation, both of which destroy dividing cells.

The term "anti-proliferative drug" is a term well understood in the art and refers to any compound that destroys dividing cells or inhibits them from undergoing further proliferation. Anti-proliferative drugs include, but are not limited to, mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxorubicin, bleomycin, plicamycin, mitomycin, L-asparaginase, cisplatin, mitoxantrone, hydroxyurea, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin and pseudomonas exotoxin A.

The agent can be administered as standard dosages as used in the art. In one embodiment, the agent is administered as a single bolus injection. In another embodiment, the agent is administered by infusion over a period of, for example, 24 hours.

### Timing of Exposing the Subject to the Agent

As outlined above, the present invention relies on the observation that the relative number of effector cells expands in response to an antigen before the regulator cells. Accordingly, as used herein, the term "the activity of the effector cells is not significantly reduced" means that the timing of the administration of the agent is such that the agent exerts a proportionally greater effect against the regulator cells than the effector cells. It is clearly preferred that the agent is administered at a time where the ratio of effect against the regulator cells to the effect against effector cells is greatest.

It has been reported that after the initial fall in viral load following anti-retroviral treatment, for instance in subjects infected with HIV, there is an increase in viral load and a subsequent stimulation of the immune response to the increase in viral load upon withdrawal of treatment (Oritz et al., 1999; Kilby et al., 2000; Lifson et al., 2000). Accordingly, the exposure of the subject to anti-viral therapy followed by removal of the therapy can be used to increase the number of, and/or activate, effector cells directed against the retrovirus enabling a targetable regulator cell expansion.

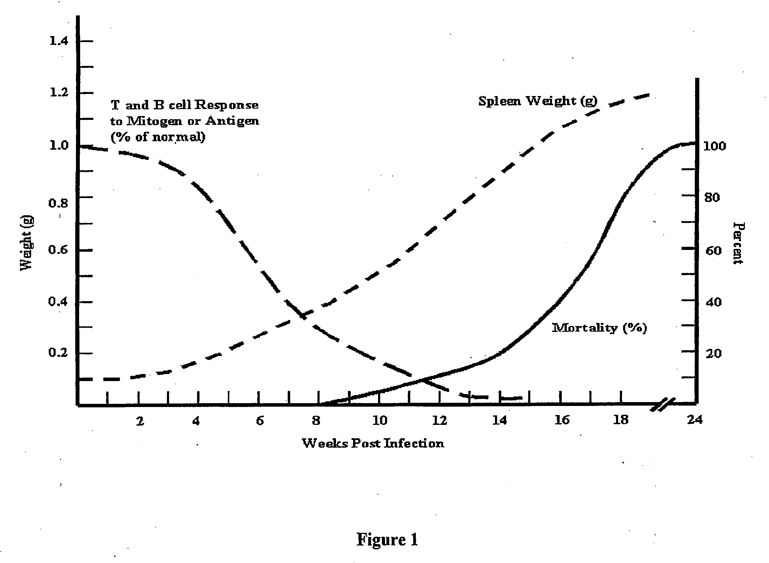

An example of the appropriate time for administering the agent can be determined by reference to Daar et al. (1998). This document provides the viral load, and CD8+ and CD4+ T cell levels, from a patient who is taken off highly active antiretroviral therapy (also known as HAART). After the initial rise in viral load, a reduction in viral load occurs when the CD8+ T cells have reached maximum numbers, and therefore effect (see Figure 1 of Daar et al., 1998). Immediately beyond this time point (a few days) CD8+ T cells start to drop off and viral load begins to stabilize after the decline.

Similarly, Figure 18 shows an initial increase in viral load in a patient upon conclusion of HAART, followed by a decrease in HIV RNA as a result of effector cell activity, which in turn is followed by another increase in HIV RNA levels as a result of regulation of the effector cells.

The peaks can be used as an inference point to predict the clonal expansion of the subsequent regulator cells and therefore an intervention point for regulator cell ablation. The agent should be applied when the majority of regulators are in clonal expansion (mitosis), which generally corresponds to when the viral load has stabilized following an initial increase and/or peaked following an initial increase, and/or possibly viral load decreases following an initial increase (as seen in Figure 18), and/or at the latest at the very beginning of a second elevation viral load.

In most instances, the time point that the agent is to be administered will need to be empirically determined in subjects at different stages of infection as their immune response kinetics may vary. Other factors such as the general health of the subject and/or the genetic makeup of the subject will also impact upon when is the appropriate time to administer the agent.

It has been established that c-reactive protein levels increase at approximately the same time as viral load increases (see Figure 18). This is followed by a decrease in c-reactive protein levels as a result of effector cell activity upon the viral load reducing retroviral numbers. Accordingly, this information can be used to determine the correct timing of administration of the agent. Thus, in a preferred embodiment, the agent is administered approximately when the levels of c-reactive protein have peaked and begun to decrease.

Another avenue of determining the time point for administering the agent is to monitor the viral load following stimulation of the immune response. Since c-reactive protein assays are relatively simple and sensitive, and the levels of c-reactive protein correlates with viral load (see Figure 18), such assays can be used to determine when to begin close monitoring of the viral load. For instance, when c-reactive protein levels have begun to increase, monitoring of the viral load could commence.

These methods described above in relation to c-reactive protein can be performed using other positive acute phase inflammatory markers.

It is envisaged that the viral load decreases due to the activity of the effector cells, however, the subsequent increase in regulator cells would down-regulate the effector cells resulting in a slowing of the viral load decrease. Accordingly, the agent could be administered prior to the slowing of the decrease in viral load. Techniques known in the art, for example RT-PCR, could be used to monitor viral load in these circumstances.

Monitoring may need to be very frequent, for example as often as every few hours, to ensure the correct time point is selected for administration of the agent. Preferably, the monitoring is conducted at least every 48 hours. More preferably, the monitoring is conducted at least every 24 hours.

Optimally, the monitoring is continued to determine the affect of the agent. Insufficient ablation, re-emergence of the regulator cells or increases in viral load within, for example, about 7 days of treatment will mean that the method or uses of the present invention should be repeated. Such repeated cycles of treatment may generate immunological memory. It is therefore possible that the present invention, used in repetitive mode, may provide some prophylactic protective effect.

### Acute Phase Inflammatory Markers

As mentioned above, some acute phase inflammatory markers initially increase during an immune response (referred to hereinafter as positive acute phase inflammatory markers) whilst others initially decrease during an immune response (referred to hereinafter as negative acute phase inflammatory markers). Acute phase inflammatory markers are also referred to in the art as acute phase reactants or acute phase proteins.

Examples of positive acute phase inflammatory markers include, but are not limited to, c-reactive protein, serum amyloid A, serum amyloid P component, complement proteins such C2, C3, C4, C5, C9, B, C1 inhibitor and C4 binding protein, fibrinogen, von Willebrand factor, α1-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein. Example of negative acute phase inflammatory markers include, but are not limited to, albumin, pre-albumin, transferin, apoAI, apoAII, α2 HS glycoprotein, inter-α-trypsin inhibitor, histidine-rich glycoprotein.

Serum amyloid A levels can be determined as known in the art, see for example O'Hara et al. (2000).

C-reactive protein (CRP) is an important positive acute phase response protein, and its concentration in serum may increase as much as 1,000-fold during the acute

phase response. CRP is a pentamer consisting of five identical subunits, each having a molecular weight of about 23,500.

C-reactive protein levels can be determined using techniques known in the art, these include, but are not limited to, those disclosed in Senju et al. (1983), Price et al. (1987) and Eda et al. (1998).

### HAART

The term "HAART" is intended to cover any combination therapy with at least three antiretroviral agents, each of which is administered to the subject in a therapeutically effective amount. For purposes of the present invention, antiretroviral agents include any substance that can inhibit, reduce, or eliminate retroviral infection of a cell. A number of these agents are commercially available for administration according to the manufacturer's recommended dosage. Such antiretroviral agents include, but are not limited to, the two classes known as reverse transcriptase inhibitors and protease inhibitors, as well as agents that are inhibitors of viral entry. Although any combination of three or more of these agents can be used, preferably HAART comprises the administration of therapeutically effective amounts of at least one reverse transcriptase inhibitor and at least one protease inhibitor in combination with at least one additional antiretroviral agent.

A number of reverse transcriptase inhibitors are commercially available for use in administering HAART. Examples include, but are not limited to, zidovudine (AZT) available under the RETROVIR tradename from Glaxo-Wellcome Inc., Research Triangle, NC 27709; didanosine (ddl) available under the VIDEX tradename from Bristol-Myers Squibb Co., Princeton, NJ 08543; zalcitabine (ddC) available under the HIVID tradename from Roche Pharmaceuticals, Nutley, N.J. 07110; stavudine (d4T) available under the ZERIT trademark from Bristol-Myers Squibb Co., Princeton, N.J. 08543; lamivudine (3TC) available under the EPIVIR tradename from Glaxo-Wellcome Research Triangle, N.C. 27709; abacavir (1592U89) disclosed in WO96/30025 and available under the ZIAGEN tradename from Glaxo-Wellcome Research Triangle, N.C. 27709; adefovir dipivoxil [bis(POM)-PMEA] available under the PREVON tradename from Gilead Sciences, Foster City, Calif. 94404; lobucavir (BMS-180194), a nucleoside reverse transcriptase inhibitor disclosed in EP-0358154 and EP-0736533 and under development by Bristol-Myers Squibb, Princeton, N.J. 08543; BCH-10652, a reverse transcriptse inhibitor (in the form of a racemic mixture of BCH-10618 and BCH-10619) under development by Biochem Pharma, Laval, Quebec H7V, 4A7, Canada; emitricitabine [(-)-FTC] licensed from Emory University under Emory Univ. US 5,814,639 and under development by Triangle Pharmaceuticals, Durham, N.C. 27707; beta-L-FD4(also called beta-L-D4C and named beta-L-2', 3'-dicleoxy-5-fluorocytidene) licensed by Yale University to Vion Pharmaceuticals, New Haven Conn. 06511; and DAPD, the purine nucleoside, (-)-beta-D-2,6,-diaminopurine dioxolane disclosed in EP 0656778 and licensed by Emory University and the University of Georgia to Triangle Pharmaceuticals, Durham, N.C. 27707; and lodenosine (FddA), 9-(2,3-dideoxy-2-fluoro-b-D-threo-pentofuranosyl)adenine, a acid stable purine-based reverse transcriptase inhibitor discovered by the NIH and under development by U.S. Bioscience Inc., West Conshohoken, Pa. 19428.

Examples of protease inhibitors useful in the present invention include, but are not limited to, saquinavir (Ro 31-8959) available in hard gel capsules under the INVIRASE tradename and as soft gel capsules under the FORTOUASE tradename from Roche Pharmaceuticals, Nutley, N.J. 07110-1199; ritonavir (ABT-538) available under the NORVIR tradename from Abbott Laboratories, Abbott Park, Ill. 60064; indinavir (MK-639) available under the CRIXIVAN tradename from Merck & Co., Inc., West Point, Pa. 19486-0004; nelfnavir (AG-1 343) available under the VIRACEPT tradename from Agouron Pharmaceuticals, Inc., LaJolla, Calif.92037-1020; amprenavir (141W94), a non-peptide protease inhibitor under development by Vertex Pharmaceuticals, Inc., Cambridge, Mass. 02139-4211 and available from Glaxo-Wellcome, Research Triangle, N.C. under an expanded access program; lasinavir (BMS-234475) available from Bristol-Myers Squibb, Princeton, N.J. 08543 (originally discovered by Novartis, Basel, Switzerland (CGP-61755); DMP-450, a cyclic urea discovered by Dupont and under development by Triangle Pharmaceuticals; BMS-2322623, an azapeptide under development by Bristol-Myers Squibb, Princeton, N.J. 08543 as a 2nd-generation HIV-1 PI; and ABT-378 under development by Abbott, Abbott Park, Ill. 60064; and AG-1549 an orally active imidazole carbamate discovered by Shionogi (Shionogi #S-1153) and under development by Agouron Pharmaceuticals, Inc., LaJolla Calif. 92037-1020.

Suitable human dosages for these compounds can vary widely. However, such dosages can readily be determined by those of skill in the art. Therapeutically effective amounts of these drugs are administered during HAART. By "therapeutically effective amount" is intended an amount of the antiretroviral agent that is sufficient to decrease the effects of HIV infection, or an amount that is sufficient to favorably influence the pharmacokinetic profile of one or more of the other antiretroviral agents used in the HAART protocol. By "favorably influence" is intended that the antiretroviral agent, when administered in a therapeutically effective amount, affects the metabolism of one or more of the other antiretroviral agents used in HAART, such that the bioavailability of the other agent or other agents is increased.

Guidance as to dosages for any given antiretroviral agent is available in the art and includes administering commercially available agents at their recommended dosages.

### Antigenic Polypeptides

As used herein, an "antigenic" polypeptide is any polypeptide sequence that contains an epitope which is capable of producing an immune response against the retrovirus. Typically, the antigenic polypeptide will comprise a sequence which is highly conserved in most retroviral isolates. However, it is envisaged that a particular retrovirus infecting an individual could be characterized and an antigenic polypeptide produced which matches the sequences of the isolate to maximise the possibility of an effective immune response.

Information regarding HIV antigens such as gp120 and other candidates can be found in Stott et al (1998).

The antigenic polypeptides can be provided in any manner known in the art which leads to an immune response. Antigenic polypeptides can be, for example, native, recombinant or synthetic. Such antigenic polypeptides include, but are not limited to, viral proteins that are responsible for attachment to cell surface receptors to initiate the infection process such as envelope glycoproteins.

Native antigenic polypeptides can be prepared, for example, by providing attenuated retrovirus, heat inactivated retrovirus or any other killed retrovirus.

The antigenic polypeptides can be provided as isolated polypeptides in a vaccine composition. In this instance the polypeptide can be purified from retroviral infected cells, expressed and isolated from recombinant cells, or synthetically produced using a peptide synthesizer.

### Vaccines

Vaccines may be prepared from one or more retroviral polypeptides. The preparation of vaccines which contain an antigenic polypeptide is known to one skilled in the art. Typically, such vaccines are prepared as injectables, or orals, either as liquid solutions or suspensions; solid forms suitable for solution in, or suspension in, liquid prior to injection or oral consumption may also be prepared. The preparation may also be emulsified, or the protein encapsulated in liposomes. The antigenic polypeptides are often mixed with carriers/excipients which are pharmaceutically acceptable and compatible with the active ingredient. Suitable carriers/excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine.

As used herein, the term "adjuvant" means a substance that non-specifically enhances the immune response to an antigenic polypeptide. Examples of adjuvants which may be effective include but are not limited to: N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. Further examples of adjuvants include aluminum hydroxide, aluminum phosphate, aluminum potassium sulfate (alum), bacterial endotoxin, lipid X, *Corynebacterium parvum* (*Propionobacterium acnes*), *Bordetella pertussis,* polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

The proportion of antigenic polypeptide and adjuvant can be varied over a broad range so long as both are present in effective amounts. For example, aluminum hydroxide can be present in an amount of about 0.5% of the vaccine mixture (Al₂O₃ basis). Conveniently, the vaccines are formulated to contain a final concentration of antigenic polypeptide in the range of from 0.2 to 200 µg/ml, preferably 5 to 50 µg/ml, most preferably 15 µg/ml.

After formulation, the vaccine may be incorporated into a sterile container which is then sealed and stored at a low temperature, for example 4°C, or it may be freeze-dried. Lyophilisation permits long-term storage in a stabilised form.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

### DNA Vaccination

DNA vaccination involves the direct *in vivo* introduction of DNA encoding an antigen into tissues of a subject for expression of the antigen by the cells of the subject's tissue. Such vaccines are termed herein "DNA vaccines" or "nucleic acid-based vaccines". DNA vaccines are described in US 5,939,400, US 6,110,898, WO 95/20660 and WO 93/19183. The ability of directly injected DNA that encodes an antigen to elicit a protective immune response has been demonstrated in numerous experimental systems (see, for example, Conry et al., 1994; Cardoso *et al.,* 1996; Cox et al., 1993; Davis et al., 1993; Sedegah et al., 1994; Montgomery et al., 1993; Ulmer et al., 1993; Wang et al., 1993; Xiang et al., 1994; Yang et al., 1997).

To date, most DNA vaccines in mammalian systems have relied upon viral promoters derived from cytomegalovirus (CMV). These have had good efficiency in both muscle and skin inoculation in a number of mammalian species. A factor known to affect the immune response elicited by DNA immunization is the method of DNA delivery, for example, parenteral routes can yield low rates of gene transfer and produce considerable variability of gene expression (Montgomery et al., 1993). High-velocity inoculation of plasmids, using a gene-gun, enhanced the immune responses of mice (Fynan et al., 1993; Eisenbraun et al., 1993), presumably because of a greater efficiency of DNA transfection and more effective antigen presentation by dendritic cells. Vectors containing the nucleic acid-based vaccine use in the invention may also be introduced into the desired host by other methods known in the art, e.g., transfection, electroporation, microinjection, transduction, cell fusion, DEAE dextran, calcium phosphate precipitation, lipofection (lysosome fusion), or a DNA vector transporter.

### Vaccines Derived from Transgenic Plants

Transgenic plants producing a retroviral antigenic polypeptide can be constructed using procedures well known in the art. A number of plant-derived edible vaccines are currently being developed for both animal and human pathogens (Hood and Jilka, 1999). Immune responses have also resulted from oral immunization with transgenic plants producing virus-like particles (VLPs), or chimeric plant viruses displaying antigenic epitopes (Mason *et al.,* 1996; Modelska *et al.,* 1998; Kapustra *et al.,* 1999; Brennan *et al.,* 1999). It has been suggested that the particulate form of these VLPs or chimeric viruses may result in greater stability of the antigen in the stomach, effectively increasing the amount of antigen available for uptake in the gut (Mason *et al.* 1996, Modelska *et al.* 1998).

### EXAMPLES

### Example 1

In order to demonstrate the present invention a murine AIDS model was used.

A murine AIDS (MAIDS) pathology induced by LP-BM5 murine leukemia virus (MuLV) in susceptible mice is an effective tool to investigate mechanisms of retrovirus-induced immunodeficiency. The MAIDS animal model displays a number of features of human AIDS. Infection of a susceptible strain such as C57BL/6 mice with LP-BM5 leads to chronic splenomegaly, hypergammaglobulinaemia and development of immunodeficiency in both T and B cells. *In vitro,* there is a progressive impairment in the responsiveness of T-cells and B-cells to mitogenic or specific antigenic stimuli. *In vivo,* infected mice become increasingly susceptible to challenge with a variety of opportunistic organisms and can develop B-cell lymphoma. Deaths are first observed at 8-10 weeks post-infection (pi), and all mice die within 24 weeks (Figure 1). These alterations in immune function reflect complex changes in the phenotype and function of all components of the immune network.

### 1. Therapeutic Effects of a Single Dose of Vinblastine Administered at Different Times Post Infection

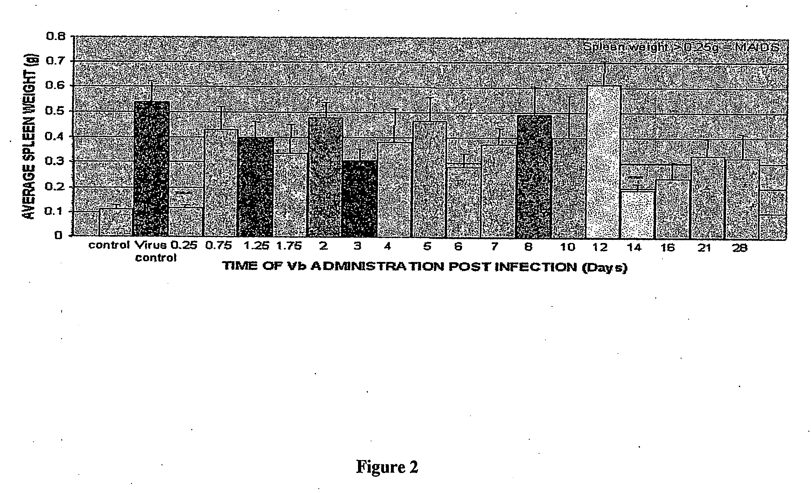

The treatment with a single dose of the anti-mitotic agent vinblastine (Vb) at different times pi is able to prevent the development/progression of MAIDS. Figure 2 shows the effect on MAIDS development (represented by average spleen weight at 10 weeks pi) of a single 6mg/kg i.p. dose of Vb given at various times pi ranging from 6hr to 28 days. Clearly, the Vb treatment is remarkably therapeutic when administered at 6 hr or 14 days pi with 100% (10/10) and 74% (20/27) of mice, respectively, showing no signs of MAIDS development at 10 weeks pi (as determined by spleen weight, histology and FACS analysis). A similar protective effect was observed in some mice treated at 6 days (6/13 or 46%) pi. Additional experiments have provided results of 100% of mice having no signs of MAIDS development at least 12 months after Vb treatment 14 days pi (data not shown). Although not as pronounced as the protection seen after day 14 treatment, moderate protection from MAIDS development was still observed. However, it is also important to note that treatment at many other time points, such as day 2 and day 7 pi, resulted in no protection against MAIDS development. The total protection from MAIDS development seen in mice given Vb at 6 hrs pi is not unexpected as the virus replicates in actively replicating cells which are the target of the anti-mitotic drug thus any cells infected with the virus are killed by the Vb preventing the virus from rapidly establishing an infection in the host. However, the highly protective effect observed after a single treatment with Vb at 14 days pi is quite remarkable as, by this stage, the viral infection is well established and the early disease processes are well underway. We propose that this highly therapeutic effect is due to the Vb targeting a particular subset of immune regulator cells during their expansion phase and thereby altering the immune response to the viral infection. The down-regulation of immune effector cells is removed by the Vb treatment targeting the regulator cells, thus allowing more effective and possibly even complete clearance of the virus by the effector cells of the immune system. This leads to long term disease free survival for many months post-infection and treatment.

### 2. Further Characterisation of the Day 14 Therapeutic Effect

### 2.1. Spleen histology in Vb treated mice at 10 weeks post infection

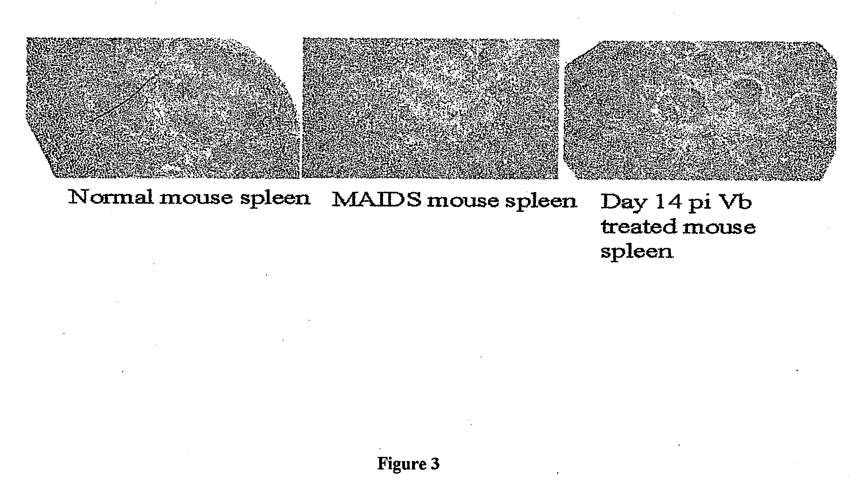

Spleens from the Day 14 pi Vb treated mice as well as infected and uninfected control mice were weighed and examined histologically. The splenic architecture of all MAIDS infected mice was profoundly disorganised as previously reported (Hartley *et al.,* 1989). In contrast, the splenic architecture of the Vb treated mice with normal spleen weights (below 0.25g) was indistinguishable from that of uninfected control mice (Figure 3).

In support of this histological data, preliminary results based on FACS analysis of spleen cells from day 14 pi Vb treated mice at 10 weeks pi showed cellular proportions (CD4⁺, CD8⁺ and B cells) and distributions like those observed in normal uninfected mice (data not shown).

### 2.2. Long-term protection from MAIDS development following Vb therapy

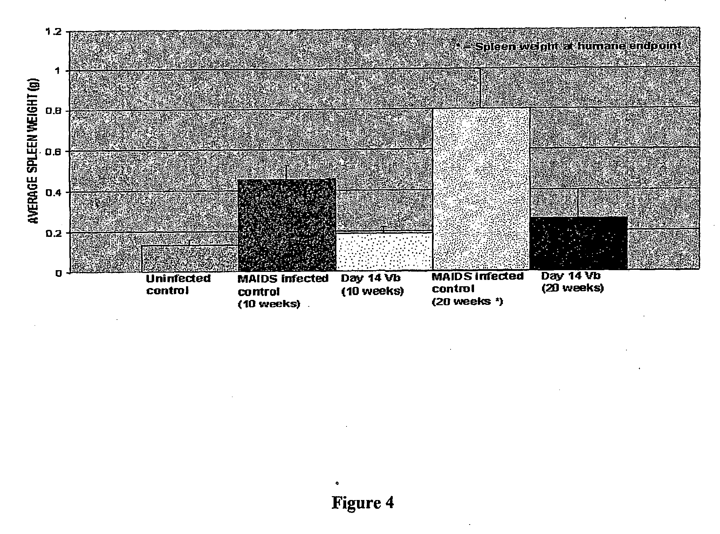

In order to determine if the infected mice were fully protected from MAIDS development or if the Vb treatment was merely delaying the disease onset and progression we treated mice with Vb at day 14 pi and waited until 20 weeks pi to examine the mice. The results of this experiment (Figure 4) showed that 80% (4/5 mice) were protected from any splenomegaly at 20 weeks pi confirming the highly effective action of the day 14 Vb treatment regime.

### 2.3. No protection from virus rechallenge following Vb therapy

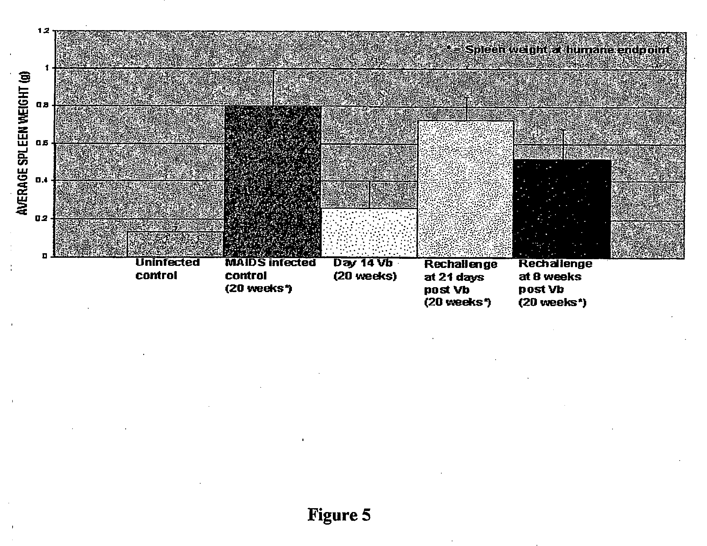

To determine if mice protected from MAIDS by the day 14 pi Vb treatment develop an immune response which would then protect them from a subsequent rechallenge with the MAIDS virus groups of mice, given the Vb therapy at day 14 pi, were rechallenged with the MAIDS virus at 3 or 8 weeks post Vb treatment. The mice were not protected from viral rechallenge and developed splenomegaly and lymphadenopathy indicating MAIDS development at 20 weeks (Figure 5). This is not an unexpected result as the dominant immunosuppression by the regulator population of cells would have been restored as a consequence of the immune effector response to the second (untreated) infection.

### 3. Vb Therapy Targets CD4⁺ cells

### 3.1. Direct FACS Analysis of Spleen Cells Following Vb Therapy on Day 14 Post Infection

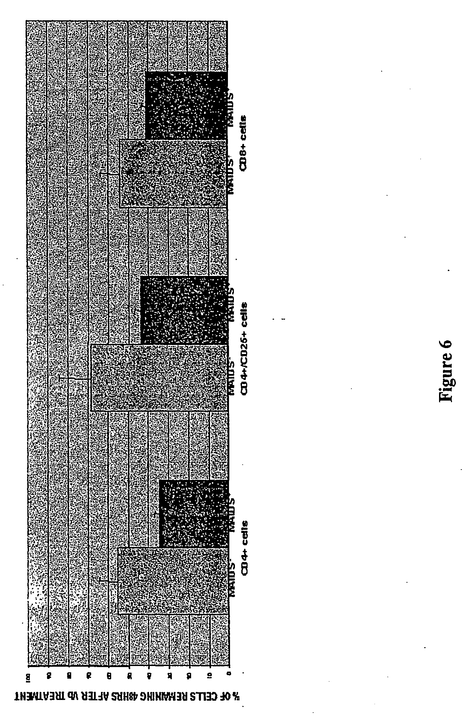

FACS analysis of CD4⁺ and CD8⁺ T cells prepared from spleens of mice treated with Vb on Day 14 pi was performed (Figure 6). CD25⁺CD4⁺ T cells were also examined as they have recently been identified as having an important regulatory function in mouse models of autoimmune disease and tumour immunology (Takahashi *et al.,* 1998; Shimizu et al., 1999). It is possible that these cells may play a role in the regulation of the immune response to MAIDS. Day 14 pi and uninfected control mice were given Vb therapy and the spleens collected at 48 hours post Vb treatment. Analysis of the data showed that while all cell subsets are reduced by the Vb therapy, comparison of the uninfected to infected ratios showed that it is the CD4⁺ T cells and CD25⁺CD4⁺ T cells that are preferentially targeted by the Vb treatment in MAIDS infected mice.

### 3. 2. Spleen cell transfer experiments: Vb therapy targets CD4⁺ cells

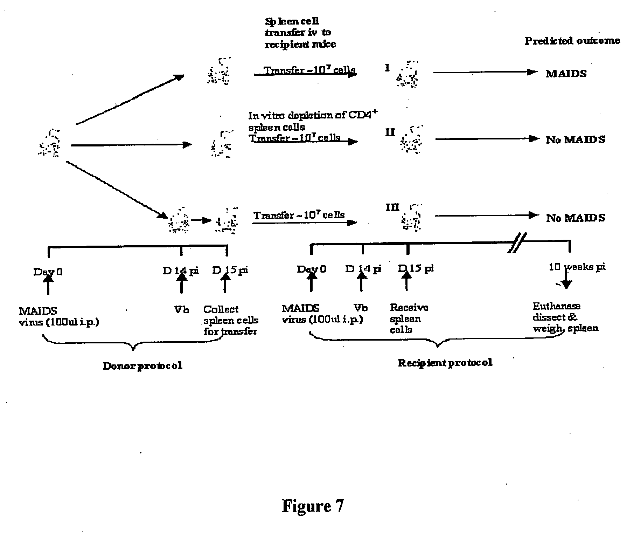

The previous experiment showed that major immune cell subsets (CD4⁺ and CD8⁺ cells) resident in the spleen are all affected by Vb treatment, however, it appears that CD4⁺ cells are preferentially targeted indicating that a subset of these cells may be clonally expanding at day 14 pi. The experiments illustrated in Figure 7 were designed to determine if a single dose of Vb, administered at 14 days pi, is eliminating a population of expanding CD4⁺ regulator T cells and thereby releasing effector cells from down-regulation and clearing the host of the MAIDS virus.

Mice were infected with MAIDS, received day 14 pi Vb treatment and then received an adoptive transfer of ∼10⁷ spleen cells from donor mice prepared as outlined in Figure 7. For each experiment [designated (I), (II) and (III)] groups consisted of 7 recipient mice and 9 donor mice. Mice in Group I received whole spleens from infected mice that had not received any Vb treatment. Mice in Group II received whole spleen cell preparations, from infected mice, that had been depleted of CD4⁺ cells using a FACS cell sorter after staining with fluorochrome-labeled anti-CD4 monoclonal antibody. Cell sorting resulted in removal of 99.5% of CD4⁺ cells from the spleen cell preparations. Mice in Group III received whole spleens from infected donor mice that also received Vb treatment on day 14 pi.

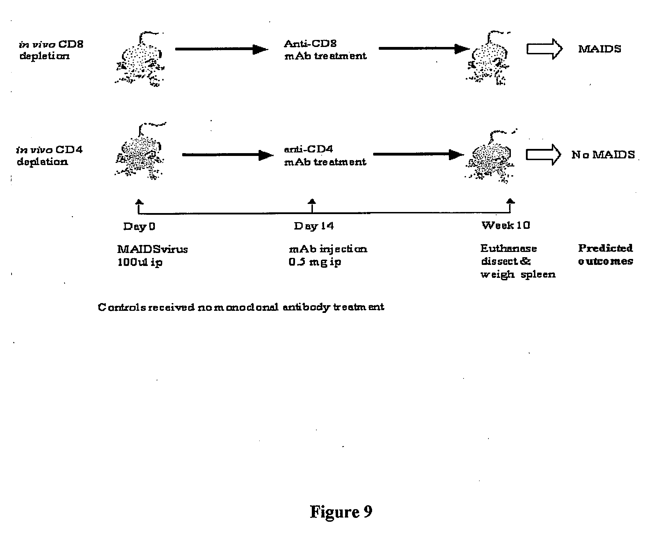

Infusing Vb-treated MAIDS infected mice with spleen cells from MAIDS infected donors completely prevented Vb from protecting mice from MAIDS development (Figure 8). Moreover, the spleen cells that overcame the protective effect of Vb were CD4⁺ cells because when CD4⁺ cells were removed from the donor spleen cells prior to transfer, by FACS sorting, the protective effect of Vb was not overcome. In addition, infusion of Vb-treated MAIDS infected mice with spleen cells from MAIDS infected donor mice that had also been given day 14 Vb showed these mice to be fully protected from MAIDS development (Figure 8).

### 3.3. In vivo depletion of CD4⁺ cells at day 14 post infection results in protection from MAIDS development

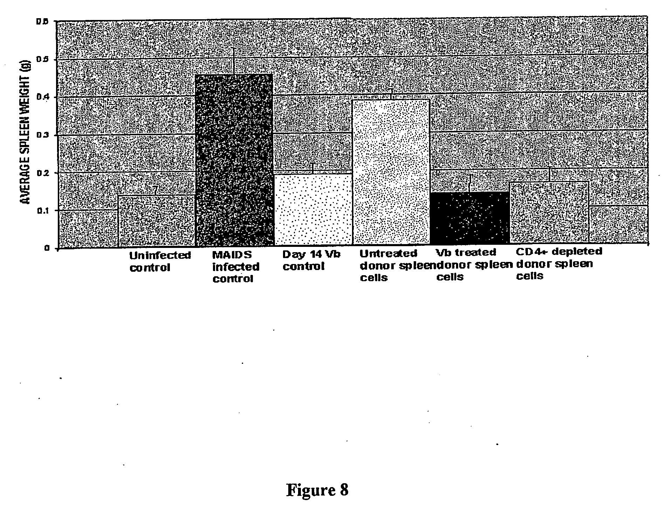

The spleen transfer results are consistent with the interpretation that, at day 14 pi, immune effector cells co-exist with a clonally expanding population of regulator cells which are CD4⁺, and that Vb is therapeutic by virtue of its ability to destroy the latter. Therefore, an *in vivo* depletion of CD4⁺ cells at the 14 day pi time point should mimic the effect of Vb. A flow diagram of the experiment is presented in Figure 9. Groups of 5 mice were infected with the MAIDS virus and then treated with monoclonal antibodies to deplete the host of either the CD4⁺ or CD8⁺ T cell subset at day 14 pi. Monoclonal antibodies were collected from the supernatants of antibody-producing hybridoma cell lines and purified by ammonium sulphate precipitation. Testing *in vivo* resulted in a 98% reduction in CD4⁺ cells and a 95% reduction in CD8⁺ cells from a single injection (0.5mg i.p.) of the appropriate concentrated monoclonal antibody preparation.

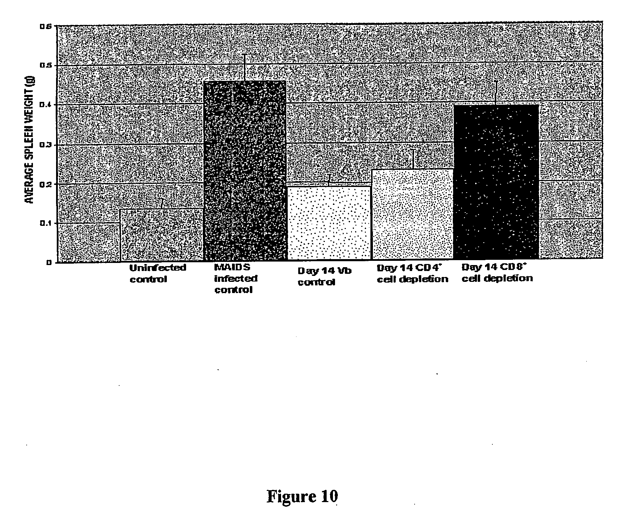

Clearly, treatment at day 14 pi with the anti-CD4⁺ monoclonal antibody resulted in prevention of MAIDS development in infected mice thereby mimicking the effect of a Vb injection at day 14 pi (Figure 10). In contrast, *in vivo* depletion of CD8⁺ cells at day 14 pi had no effect on disease progression. This result further supports the hypothesis that a population of CD4⁺ T cells is functionally down-regulating immune effector cells responding to the viral infection. In addition, the removal of the dominant CD4⁺ regulator cells enables the immune effector cells to respond more effectively to the viral infection resulting in far slower disease progression or perhaps complete clearance of the MAIDS virus from its host.

### 4. Role of CD4+CD25+ T Cells as Regulator Cells

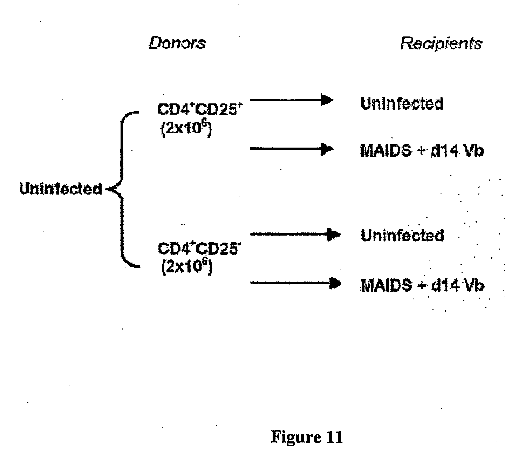

An adoptive transfer experiment was designed to directly test the hypothesis that CD4+CD25+ T cells are functioning as the regulator cells in the murine AIDS model. The same approach used to determine that the regulator cells are CD4+ T cells was used to test whether the CD4+CD25+ subset are the target of day 14 Vinblastine treatment. Figure 11 shows the overall rationale behind the experiment. 2.0x10⁶ cells of the regulator CD4+CD25+ phenotype were purified from single cell suspensions of donor spleens using MACS beads (Miltenyi Biotec) using two positive selection steps. The cells were then tested for purity by flow cytometry (both CD4+CD25+ and CD4+CD25- fractions were of > 90 % purity) and viability using trypan blue exclusion prior to transfer into uninfected (control) and MAIDS infected day 14 Vinblastine treated mice. CD4+CD25- cells were also transferred to allow that the regulator cells may not present in the CD25+ fraction and also to allow for the possibility that these putative regulator cells may lose CD25 expression as they expand and become activated as was reported by Gavin *et al.,* 2002.

Mice receiving adoptive transfer of cells were either uninfected (control) or MAIDS infected and day 14 Vinblastine treated to examine whether the transfer of cells would result in disease progression. Basically, if the CD4+CD25+ T cells are functioning as the regulator cells then the restoration of these cells via adoptive transfer should result in disease progression, abrogating the protective effect of day 14 Vinblastine treatment. Disease progression was assessed by spleen weight at 10 weeks post MAIDS infection.

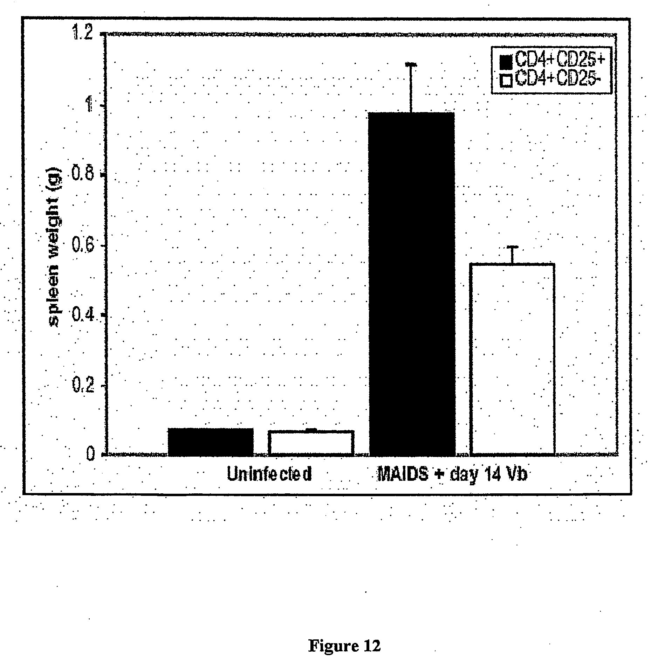

The results of the adoptive transfer experiment using uninfected donor mice are shown in Figure 12. In the negative control experiment the transfer of uninfected donor cells of CD4₊CD25_{+/-} phenotype did not result in any change in the 10 weeks pi spleen weight of uninfected recipients. However, when the CD4+CD25++/ cells were transferred in MAIDS-infected day 14 Vb-treated recipients disease progression was readily apparent. Interestingly there is a statistically significant difference in the spleen weights of these two groups (*p*=0.033) indicating that disease progressed more markedly in the group receiving the CD4+CD25+ cells. Indeed, control MAIDS spleens at 10 weeks pi are about 0.5-0.6g in these experiments. These results suggest that the regulator cells exist in both the CD4+CD25+ and the CD25- fractions, with the CD25+ portion being the more active in suppressing effector cells.

### 5. Cytokine Levels Following MAIDS Infection

### 5. 1 Serum ELISA

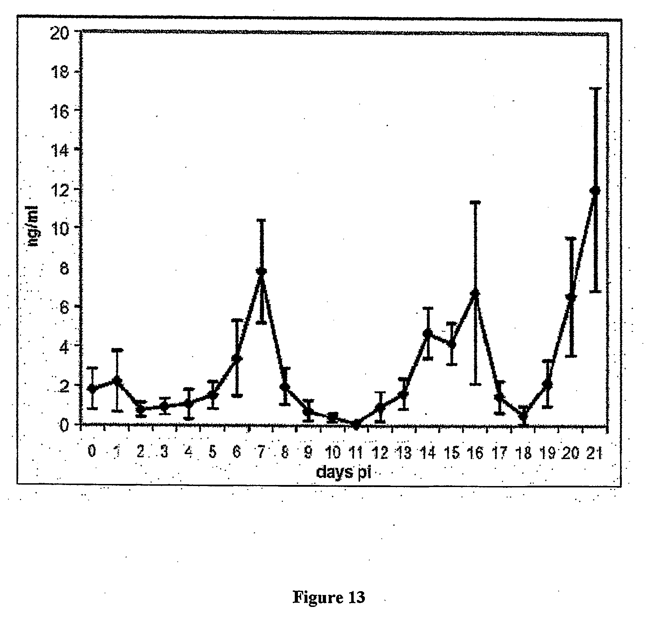

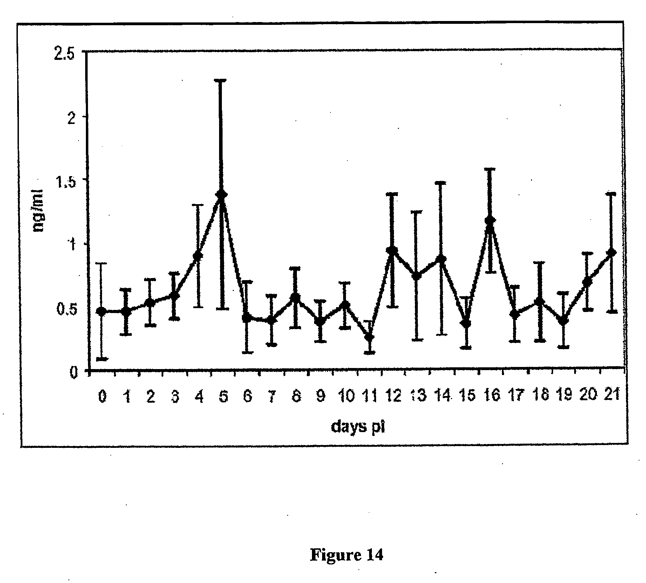

Quantitative ELISA for the presence of IL-4 and IL-10 in the serum of mice in the first three weeks post infection has been conducted. IL-4 and IL-10 are both cytokines that have been documented as being required for the maturation of and produced by regulator cells (Gavin *et al,* 2002, McGuirk and Mills, 2002). Briefly, blood was obtained by tail bleeds of mice at the different days post infection and centrifuged at high speed to collect the serum. These samples were then assayed for cytokine levels and the resultant data is shown in Figures 13 and 14.

There are two clear peaks of IL-4 production, the first at 7 days pi and the second at 14-16 days pi before increasing beyond day 21 pi. The final increase is probably associated with the B cell oligoclonal expansions that are the dominant feature of MAIDS in its later stages.

The same serum samples were also assayed for the presence of IL-10. IL-10 also displayed a three-peaked pattern with an early peak at day 4-5 pi the second at days 12-14 and the third at day 16 pi. It is interesting to note that a peak in the percentage of CD4+CD25+ cells occurred on days 10-12 (data not shown), overlapping the second IL-10 peak. Notably, CD25 (i.e. the IL-2 receptor) is expressed in a transient fashion prior to the cell population entering the cell cycle and mitosis (Roitt et al. 1989). The combined pattern is suggestive of a small regulator cell population expanding and becoming functional at precisely the time when Vb or anti-CD4 treatment ablates MAIDS progression. That is to say this data is in agreement with the T cell regulation hypothesis described herein.

### 5.2 Cytokine levels by intracellular flow

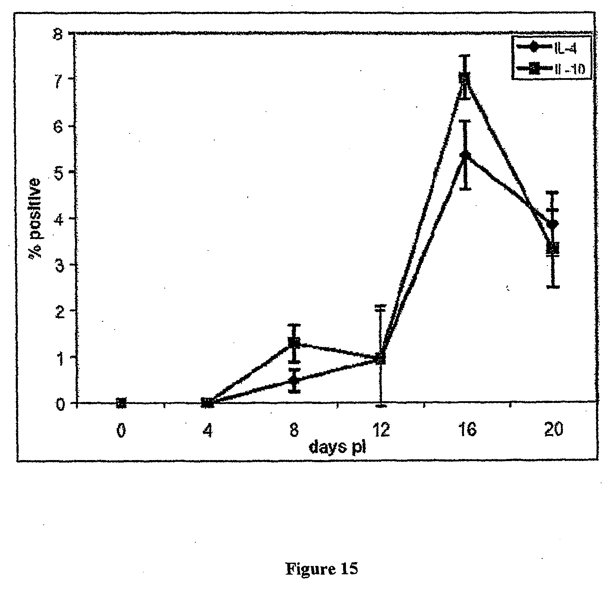

Intracellular flow is a method that has been developed and used to examine the *ex vivo* levels of cytokines present in individual cells using flow cytometry. In this experiment mice were infected with MAIDS and the spleens harvested every fourth day post infection (days 4, 8, 12, 16 and 20) as well as a day 0 uninfected control to get baseline levels of intracellular cytokine production. The spleens were prepared as single cell suspensions, the red blood cells lysed and the WBCs then fixed, permeabalised and stained for a combination of cell surface markers and cytokines. The results of the experiment are shown in Figure 15.

The expression levels of IL-4 and IL-10 are very similar with both peaking at day 16 pi and then decreasing by day 20 pi, but not returning to baseline levels. This data is in agreement with the results of the serum ELISAs (Figures 13 and 14) where a peak in the production of both cytokines was observed at day 16 pi and, as previously noted, correlates well with the CD4+CD25+ percentage profile post MAIDS infection.

### 5.3 Cytokine levels by ELISpot

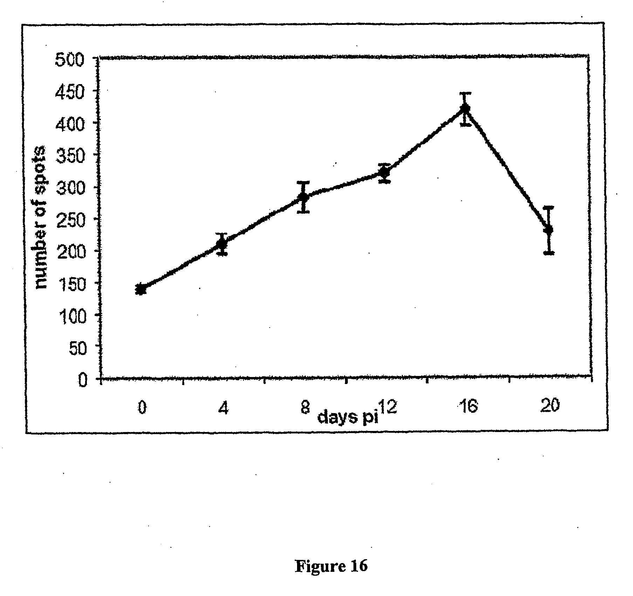

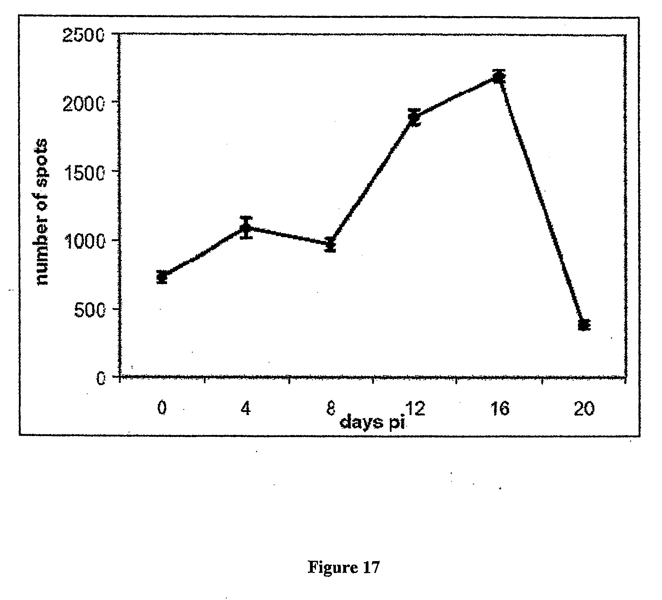

ELISpot is an ELISA-based technique that allows cytokine production to be analysed on a single cell level. The cells of interest are incubated in wells, the base of which is a membrane pre-coated with a monoclonal antibody for the cytokine of interest. This incubation step can be with or without a stimulant for the cells. As the plate is not moved during the incubation the secreted cytokine is localised to the position of the cell as it is captured by the antibody bound to the membrane base such that every cell that secretes cytokine leaves a spot on the membrane that is visible following antibody detection. The technique allows quantitation of the number of cells' producing a cytokine of interest down to the level of a single cell. For this experiment splenic WBCs were collected from mice at days 0, 4, 8, 12, 16 and 20 pi using the same protocol as for the intracellular flow. These cells were then incubated overnight on the IL-4 and IL-10 ELISpot plates without stimulation. The plates were then developed and the spots counted. The results for IL-4 are shown in Figure 16 and for IL-10 in Figure 17.

Again, as with the intracellular flow and serum ELISA, a similar trend is seen for both cytokines with increasing numbers of cells expressing each cytokine up to a peak at day 16 pi, followed by a marked decrease by day 20 pi.

### 6. Summary

This example indicates that there is a population of "regulator" cells that down regulate the immune response to the MAIDS virus, allowing the virus to proliferate and cause disease. Removal of the regulator cells at the appropriate times after infection allows for effector cells (such as B cells and CD8⁺ T cells) to more effectively clear the virus, preventing the development of disease. These regulator cells are proposed to control the activation and/or function of cells such as CD8⁺ T cells and B cells, which act as effector cells, to fight viral infection.

Modulation of the immune response by specifically eliminating a population of immune regulator cells has previously been investigated by Robert North and colleagues using a murine T cell lymphoma model (North and Awwad, 1990). North proposed that the immunogenic tumour was able to establish and grow due to down regulation of the immune response by a population of CD4⁺ regulator T cells. This regulation did not allow the immune response to develop sufficiently in magnitude to cause tumour regression. It was noted that treatment with a single dose of Vinblastine (Vb) at different times following tumour inoculation resulted in enhancement (day 10) or regression (days 4, 6, 13 and 15) of the tumour. North hypothesised that the observed regression was due to elimination of the CD4⁺ regulator T cells at those time points. In a series of experiments involving the selective depletion of CD4⁺ and CD8⁺ T cells the regulator cells were identified as being of the CD4⁺ phenotype.

The chromosomal DNA of inbred and wild mice contains multiple copies of sequences reactive with MuLV nucleic acid probes. Among these sequences are complete, potentially infectious genomes of numerous MuLVs (Chattopadhyay *et al.,* 1980). All MuLVs share high sequence homology. We hypothesize that when these endogenous MuLV proteins are expressed during development they are recognised as self-antigens by the immune system. Due to the high level of homology between endogenous and exogenous MuLV nucleic and amino acid sequences an infecting MuLV may also be partly recognised as self by the immune system, and any immune response to the viral infection will be down-regulated, resulting in a lack of viral clearance and the development of disease.

### Example 2

A human subject suffering from a HIV infection was subject to HAART for at least 6 months and then taken off the treatment. Viral load and c-reactive protein levels were determined using standard techniques on samples obtained during and after the completion ofHAART.

As can be seen in Figure 18, the results show that upon conclusion of HAART viral load increased. This was followed by a decrease in viral load as a result of effector cell activity which in turn was followed by another increase in viral load resulting from regulation of the effector cells. C-reactive protein levels closely mirrored viral load indicating that assays for this protein are useful as a marker for effector cell activity, as well as viral load.

### Example 3

A human patient suffering from an HIV infection is administered with a vaccine comprising retroviral antigenic polypeptides. Examples of such vaccines are reviewed in Dennehy (2001) and Moore et al. (2001).

Following vaccine administration, c-reactive protein levels are analysed as generally described in Example 2. Preferably, c-reactive protein levels are determined at least every 24 hours. Naturally, the patient should be examined for any indications of, for example, other viral or bacterial infections which may contribute to the elevated c-reactive protein levels. In the absence of such indications, c-reactive protein levels are continued to be monitored until levels of c-reactive protein peak and begin to decrease. Approximately when the levels of c-reactive protein begin to decrease as an indication of regulation of the effector cells the subject is administered with anti-CD4+ antibodies at a standard dose such as 300mg.

The patient is then continued to be monitored for HIV markers, such as determining viral load. If there is evidence that the infection has not been suitably controlled the treatment can be repeated.

### Example 4

A human patient suffering from an HIV infection is subjected to standard HAART treatment. Following the conclusion of HAART the subjects c-reactive protein levels are analysed as generally described in Example 2. Preferably, c-reactive protein levels are determined at least every 24 hours. Approximately when the levels of c-reactive protein begin to decrease as an indication of regulation of the effector cells the subject is administered with vinblastine at a standard dose such as 3-4 mg/m² intravenously (Casciato and Lowitz, 1995). Vinblastine will target dividing cells, such as the regulator cells, which begin to clonally expand to control effector cell levels.

The patient is then continued to be monitored for HIV markers, such as determining viral load. If there is evidence that the infection has not been suitably controlled the treatment can be repeated.

### REFERENCES:

Barin, F., McLane, M.F., Allan, J.S., Lee, T.H., Groopman, J.E. and Essex, M. (1985). Virus envelope protein of the HTLV-III represents major target antigen for antibodies in AIDS patients. Science 228,1094-6.

Brennan, F.R., Bellaby, T., Helliwell, S.M., Jones, T.D., Kamstrup, S., Dalsgaard, K., Flock, J-I. and Hamilton, W.D.O. (1999). Chimeric plant virus particles administered nasally or orally induce systemic and mucosal immune responses in mice. Journal of Virology 73,930-8.

Cardoso, A.I., Blixenkrone-Moller, M., Fayolle, J., Liu, M., Buckland, R. and Wild, T.F. (1996). Immunization with plasmid DNA encoding for the measles virus hemagglutinin and nucleoprotein leads to humoral and cell-mediated immunity. Virology 225,293-9.

Casciato, D.A. and Lowitz, B.B. (1995) Manual of Clinical Oncology. Third Edition. Little Brown and Company, Boston.

Chattopadhyay, S.K., Lander, M.R., Rands, E. and Lowy, D.R. (1980). Structure of endogenous murine leukemia virus DNA in mouse genomes. Proceedings of the National Academy of Sciences of the USA 77,5774-8.

Coll, J., Palazon, J., Yazbeck, H., Gutierrez, J., Aubo, C., Benito, P., Jagiello, P., Maldyk, H., Marrugat, J. and Anglada, J. et al. (1995). Antibodies to human immunodeficiency virus (HIV-1) in autoimmune diseases: primary Sjogren's syndrome, systemic lupus erythematosus, rheumatoid arthritis and autoimmune thyroid diseases. Clinical Rheumatology 14,451-7.

Conry, R.M., LoBuglio, A.F., Kantor, J., Schlom, J., Loechel, F., Moore, S.E., Sumerel, L.A., Barlow, D.L., Abrams, S. and Curiel, D.T. (1994). Immune response to a carcinoembryonic antigen polynucleotide vaccine. Cancer Research 54,1164-68.

Cox, G.J., Zamb, T.J. and Babiuk, L.A. (1993). Bovine herpesvirus 1: immune responses in mice and cattle injected with plasmid DNA. Journal of Virology 67,5664-7.

Daar, E.S., Bai, J., Hausner, M.A., Majchrowicz, M., Tamaddon, M. and Giorgi, J.V. (1998). Acute HIV syndrome after discontinuation of antiretroviral therapy in a patient treated before seroconversion. Annals of Internal Medicine 128, 827-9.

Davis, H.L., Michel, M.L. and Whalen, R.G. (1993). DNA-based immunization induces continuous secretion of hepatitis B surface antigen and high levels of circulating antibody. Human Molecular Genetics 2,1847-51.

Dennehy, P.H. (2001). Active immunization in the United States: Developments over the past decade. Clinical Microbiology Reviews 14,872-908.

Eda, S., Kaufmann, J., Roos, W. and Phol, S. (1998). Development of a new microparticle-enhanced turbidimetric assay for c-reactive protein with superioir features in analytical sensitivity and dynamic range. Journal of Clinical Laboratory Analysis 12,137-144.

Eisenbraun, M.D., Fuller, D.H. and Haynes, J.R. (1993). Examination of parameters affecting the elicitation of humoral immune responses by particle bombardment-mediated genetic immunization. DNA and Cell Biology 12,791-7.

Fynan, E.F., Webster, R.G., Fuller, D.H., Haynes, J.R., Santoro, J.C. and Robinson, H.L. (1993). DNA vaccines: protective immunization by parenteral, mucosal, and genegun inoculations. Proceedings of the National Academy of Sciences of the USA 90,11478-82.

Gavin, M.A., Clarke, S.R., Negrou, E., Gallegos, A. and Rudensky, A. (2002). Homeostasis and anergy of CD4(+)CD25(+) suppressor T cells in vivo. Nature Immunology 3,33-41.

Hartley, J.W., Fredrickson, T.N., Yetter, R.A., Makino, M. and Morse, H.C.D. (1989). Retrovirus-induced murine acquired immunodeficiency syndrome: natural history of infection and differing susceptibility of inbred mouse strains. Journal of Virology 63,1223-31.

Hood, E.E. and Jilka, J.M. (1999). Plant-based production of xenogenic proteins. Current Opinions in Biotechnology 10,382-6.

Kapustra, J., Modelska, A., Figlerowicz, M., Pniewski, T., Letellier, M., Lisowa, O., Yusibov, V., Koprowski, H., Plucienniczak, A. and Legocki, A.B. (1999). A plant-derived edible vaccine against hepatitis B virus. FASEB Journal 13,1796-99.

Kilby, J.M., Goepfert, P.A., Miller, A.P., Gnann Jr, J.W., Sillers, M., Saag, M. and Bucy, R.P. (2000). Recurrence of the acute HIV syndrome after interruption of antiretroviral therapy in a patient with chronic HIV infection: A case report. Annals of Internal Medicine 133,435-8.

Lifson, J.D., Rossio, J.L., Arnaout, R., Li, L., Parks, T.L., Schneider, D.K., Kiser, R.F., Coalter, V.J., Walsh, G., Imming, R.J., Fisher, B., Flynn, B.M., Bischofberger, N., Piatak Jr, M., Hirsch, V.M., Nowak, M.A. and Wodarz, D. (2000). Containment of simian immunodeficiency virus infection: cellular immune responses and protection from rechallenge following transient postinoculation antiretroviral treatment. Journal of Virology 74,2584-93.

Mason H.S., Ball J.M., Shi J.J., Jiang X., Estes M.K. and Arntzen C.J. (1996). Expression of Norwalk virus capsid protein in transgenic tobacco and potato and its oral immunogenicity in mice. Proceedings of the National Academy of Sciences of the USA 93,5335-40.

McGuirk, P. and Mill, K. (2002). Pathogen-specific regulatory T cells provoke a shift in the Th1/Th2 paradigm in immunity to infectious diseases. Trends in Immunology 23,450-5.

Mitsuya, H., Yarchoan, R., Kageyama, S. and Broder, S. (1991). Targeted therapy of human immunodeficiency virus-related disease. FASEB Journal 5,2369-81.

Modelska, A., Dietzschold, B., Sleysh, N., Fu, Z.F., Steplewski, K., Hooper, D.C., Koprowski, H. and Yusibov, V. (1998). Immunization against rabies with plant-derived antigen. Proceedings of the National Academy of Sciences of the USA 95,2481-85.

Montgomery, D.L., Shiver, J.W., Leander, K.R., Perry, H.C., Friedman, A., Martinez, D., Ulmer, J.B., Donnelly, J.J. and Lui, M.A. (1993). Heterologous and homologous protection against influenza A by DNA vaccination: optimization of DNA vectors. DNA and Cell Biology 12,777-83.

Moore, J.P., Parren, P.W.H.I. and Burton, D.R. (2001). Genetic subtypes, humoral immunity, and human immunodeficiency virus type 1 vaccine development. Journal of Virology 75,5721-5729.

North, R.J. and Awwad, M. (1990). Elimination of cycling CD4+ suppressor T cells with an anti-mitotic drug releases non-cycling CD8+ T cells to cause regression of an advanced lymphoma. Immunology 71,90-5.

O'Hara, R, Murphy, E.P., Whitehead, A.S., Fitzgearld, O. and Bresnihan, B. (2000). Acute-phase serum amyloid A production by rheumatoid arthritis synovial tissue. Arthritis Research 2, 142-144.

Ortiz, G.M., Nixon, D.F., Trkola, A., Binley, J., Jin, X., Bonhoeffer, S., Kuebler, P.J., Donahoe, S.M., Demoitie, M.-A., Kakimoto, W.M., Ketas, T., et al. (1999). HIV-1-specific immune responses in subjects who temporarily contain virus replication after discontinuation of highly active antiretroviral therapy. Journal of Clinical Investigation, published online, September 1999.

Price, C.P., Trull, A.K., Berry, D. and Gorman, E.G. (1987) Development and validation of a particle-enhanced turbidimetric immunoassay for C-reactive protein. Journal of Immunological Methods 99,205-211.

Rakowicz-Szulczynska, E.M. (2000). Relevance of the viral RAK alpha gene in diagnosis of malignant versus nonmalignant tumors of the ovary and uterus. Clinical and Diagnostic Laboratory Immunology 7,360-5.

Rakowicz-Szulczynska, E.M., Jackson, B., Szulczynska, A.M. and Smith, M. (1998). Human immunodeficieny virus type 1-like DNA sequences and immunoreactive viral particles with unique association with breast cancer. Clinical and Diagnostic Laboratory Immunology 5,645-53.

Roitt, Brostoff and Male (1989) Immunology. Chapter 8, Gower Medical Publishing, London.

Sedegah, M., Hedstrom, R., Hobart, P. and Hoffman, S.L. (1994). Protection against malaria by immunization with plasmid DNA encoding circumsporozoite protein. Proceedings of the National Academy of Sciences of the USA 91,9866-70.

Senju, O., Takagi, Y., Gomi, K., Ishii, N., Mochizuki, S. Ishii, N. et al. (1983). The quantitative determination of CRP by latex agglutination photometric assay. Japanese Journal of Clinical Laboratory Automation 8, 161-165.

Shimizu, J., Yamazaki, S. and Sakaguchi, S. (1999). Induction of tumor immunity by removing CD25+CD4+ T cells: a common basis between tumor immunity and autoimmunity. Journal of Immunology 163,5211-8.

Stott, J., Hu, S.L., and Almond, N. (1998). Candidate vaccines protect macaques against primate immunodeficiency viruses. AIDS Research and Human Retroviruses. Suppl 3, S265-70.

Takahashi, T., Kuniyasu, Y., Toda, M., Sakaguchi, N., Itoh, M., Iwata, M., Shimizu, J. and Sakaguchi, S. (1998). Immunologic self-tolerance maintained by CD25+CD4+ naturally anergic and suppressive T cells: induction of autoimmune disease by breaking their anergic/suppressive state. International Immunology 10,1969-80.

Ulmer, J.B., Donnelly, J.J., Parker, S.E., Rhodes, G.H., Felgner, P.L., Dwarki, V.J., Gromkowski, S.H., Deck, R.R., DeWitt, C.M., Friedman, A. et al. (1993). Heterologous protection against influenza by injection of DNA encoding a viral protein. Science 259,1745-49.

Wang, B., Ugen, K.E., Srikantan, V., Agadjanyan, M.G., Dang, K., Refaeli, Y., Sato, A.I., Boyer, J., Williams, W.V. and Weiner, D.B. (1993). Gene inoculation generates immune responses against human immunodeficiency virus type 1. Proceedings of the National Academy of Sciences of the USA 90,4156-60.

Xiang, Z.Q., Spitalnik, S., Tran, M., Wunner, W.H., Cheng, J. and Ertl, H.C. (1994). Vaccination with a plasmid vector carrying the rabies virus glycoprotein gene induces protective immunity against rabies virus. Virology 199,132-40.

Yang, K., Mustafa, F., Valsamakis, A., Santoro, J.C., Griffin, D.E. and Robinson, H.L. (1997). Early studies on DNA-based immunization for measles virus. Vaccine 15,888-91.

## Claims

1. A method for determining when an agent which inhibits the production of, limits the function of and/or destroys, regulator T cells, is to be administered to a mammalian subject with a retroviral infection that has been exposed to a treatment which increases the number of, and/or activates, effector CD8+ T cells directed against the retrovirus, the method comprising analysing a sample previously obtained from the subject after the treatment for an acute phase inflammatory marker, wherein fluctuations in the levels of the acute phase inflammatory marker is used to assist in determining when the agent is to be administered, and wherein the timing of administration of the agent is selected such that it exerts a proportionally greater effect against the regulator T cells than the effector CD8+ T cells, and wherein the agent is selected from the group consisting of anti-proliferative drugs, radiation and an antibody which binds specifically to CD4+ T cells;
wherein the acute phase inflammatory marker is a positive acute phase inflammatory marker selected from the group consisting of: c-reactive protein, serum amyloid A, serum amyloid P component, complement proteins selected from C2, C3, C4, C5, C9, B, C1 inhibitor and C4 binding protein, fibrinogen, von Willebrand factor, α1-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein; or
wherein the acute phase inflammatory marker is a negative acute phase inflammatory marker selected from the group consisting of: albumin, pre-albumin, transferin, apoAI, apoAII, α2 HS glycoprotein, inter-α-trypsin inhibitor and histidine-rich glycoprotein 2.

2. The method of claim 1, wherein the acute phase inflammatory marker is a positive acute phase inflammatory marker.

3. The method of claim 2, wherein the positive acute phase inflammatory marker is c-reactive protein or serum amyloid A.

4. The method of claim 3, wherein the positive acute phase inflammatory marker is c-reactive protein.

5. The method according to any one of claims 2 to 4, wherein effector cell production following the treatment is indicated by increased levels of positive acute phase inflammatory marker.

6. The method of claim 1, wherein the acute phase inflammatory marker is a negative acute phase inflammatory marker.

7. The method of claim 6, wherein the negative acute phase inflammatory marker is albumin.

8. The method according to any one of claims 1 to 7, wherein the method comprises analysing samples obtained at least every 48 hours from the subject.

9. The method according to any one of claims 1 to 7, wherein the method comprises analysing samples obtained at least every 24 hours from the subject.

10. The method according to any one of claims 1 to 10, wherein the treatment which increases the number of, and/or activates, effector CD8+ T cells directed against the retrovirus is (i) withdrawal of antiretroviral drug therapy or (ii) a composition which increases the number of and/or activates effector cells directed against a retrovirus, and wherein the composition comprises a retroviral antigenic polypeptide or DNA encoding the polypeptide.

11. Use of a composition which increases the number of and/or activates effector cells directed against a retrovirus for the manufacture of a medicament for treating a retroviral infection in a mammalian subject, wherein the subject is subsequently administered with an agent which inhibits the production of, limits the function of, and/or destroys, regulator T cells, and wherein the timing of administration of the agent is selected such that it exerts a proportionally greater effect against the regulator T cells than the effector CD8+ T cells, and wherein fluctuations in the levels of an acute phase inflammatory marker in the subject are used to assist in determining when the agent is administered, and wherein the composition comprises a retroviral antigenic polypeptide or DNA encoding the polypeptide, and wherein the agent is selected from the group consisting of anti-proliferative drugs, radiation and an antibody which binds specifically to CD4+ T cells, wherein the acute phase inflammatory marker is a positive acute phase inflammatory marker selected from the group consisting of: c-reactive protein, serum amyloid A, serum amyloid P component, complement proteins selected from C2, C3, C4, C5, C9, B, C1 inhibitor and C4 binding protein, fibrinogen, von Willebrand factor, α1- antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein; or
wherein the acute phase inflammatory marker is a negative acute phase inflammatory marker selected from the group consisting of: albumin, pre-albumin, transferin, apoAI, apoAII, α2 HS glycoprotein, inter-α-trypsin inhibitor and histidine-rich glycoprotein.

12. Use of an agent which inhibits the production of, limits the function of, and/or destroys, regulator T cells for the manufacture of a medicament for treating a retroviral infection in a mammalian subject, wherein the subject has been previously administered with a composition which increases the number of and/or activates effector cells directed against a retrovirus, and wherein the agent is administered at a time selected such that it exerts a proportionally greater effect against the regulator T cells than the effector CD8+ T cells, and wherein fluctuations in the levels of an acute phase inflammatory marker in the subject are used to assist in determining when the agent is administered, and wherein the agent is selected from the group consisting of anti-proliferative drugs, radiation and an antibody which binds specifically to CD4+ T cells, and wherein the composition comprises a retroviral antigenic polypeptide or DNA encoding the polypeptide;
wherein the acute phase inflammatory marker is a positive acute phase inflammatory marker selected from the group consisting of: c-reactive protein, serum amyloid A, serum amyloid P component, complement proteins selected from C2, C3, C4, C5, C9, B, C1 inhibitor and C4 binding protein, fibrinogen, von Willebrand factor, α1-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein; or
wherein the acute phase inflammatory marker is a negative acute phase inflammatory marker selected from the group consisting of: albumin, pre-albumin, transferin, apoAI, apoAII, α2 HS glycoprotein, inter-α-trypsin inhibitor and histidine-rich glycoprotein.

13. The use of claim 11 or claim 12, wherein the agent is administered approximately when the number of viral particles has begun to stabilize or increase following administration of the composition, wherein testing for viral particle level in the subject begins when positive acute phase inflammatory marker levels begins to increase following administration of the composition.

14. Use of an antiretroviral drug for the manufacture of a medicament for treating a retroviral infection in a mammalian subject, wherein the subject is subsequently administered with an agent which inhibits the production of, limits the function of, and/or destroys, regulator T cells, wherein the agent is administered after the antiretroviral drug therapy has concluded and a resulting expansion in retroviral numbers has led to an increase in the number, and/or activation of, effector cells directed against the retrovirus, and wherein the timing of administration of the agent is selected such that it exerts a proportionally greater effect against the regulator T cells than the effector CD8+ T cells, and wherein fluctuations in the levels of an acute phase inflammatory marker in the subject are used to assist in determining when the agent is administered, and wherein the agent is selected from the group consisting of anti-proliferative drugs, radiation and an antibody which binds specifically to CD4+ T cells;
wherein the acute phase inflammatory marker is a positive acute phase inflammatory marker selected from the group consisting of: c-reactive protein, serum amyloid A, serum amyloid P component, complement proteins selected from C2, C3, C4, C5, C9, B, C1 inhibitor and C4 binding protein, fibrinogen, von Willebrand factor, α1-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein; or
wherein the acute phase inflammatory marker is a negative acute phase inflammatory marker selected from the group consisting of: albumin, pre-albumin, transferin, apoAI, apoAII, α2 HS glycoprotein, inter-α-trypsin inhibitor and histidine-rich glycoprotein.

15. Use of an agent which inhibits the production of, limits the function of, and/or destroys, regulator T cells for the manufacture of a medicament for treating a retroviral infection in a mammalian subject, wherein the subject has been previously administered with an antiretroviral drug, wherein the agent is administered after the antiretroviral drug therapy has concluded and a resulting expansion in retroviral numbers has led to an increase in the number, and/or activation of, effector cells directed against the retrovirus, and wherein the agent is administered at a time selected such that it exerts a proportionally greater effect against the regulator T cells than the effector CD8+ T cells, and wherein fluctuations in the levels of an acute phase inflammatory marker in the subject are used to assist in determining when the agent is administered, and wherein the agent is selected from the group consisting of anti-proliferative drugs, radiation and an antibody which binds specifically to CD4+ T cells;
wherein the acute phase inflammatory marker is a positive acute phase inflammatory marker selected from the group consisting of: c-reactive protein, serum amyloid A, serum amyloid P component, complement proteins selected from C2, C3, C4, C5, C9, B, C1 inhibitor and C4 binding protein, fibrinogen, von Willebrand factor, α1-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a) and lipopolysaccharide-binding protein; or
wherein the acute phase inflammatory marker is a negative acute phase inflammatory marker selected from the group consisting of: albumin, pre-albumin, transferin, apoAI, apoAII, α2 HS glycoprotein, inter-α-trypsin inhibitor and histidine-rich glycoprotein.

16. The use of claim 14 or claim 15, wherein the agent is administered approximately when the number of viral particles has peaked or begun to decrease after this peak, in response to the conclusion of the antiretroviral drug therapy, wherein testing for viral particle levels in the subject begins when levels of a positive acute phase inflammatory marker begins to increase following conclusion of the antiretroviral drug therapy.

17. The use according to any one of claim 14 to 16, wherein the antiretroviral drug therapy is HAART.

18. The use according to any one of claims 11 to 17, wherein the acute phase inflammatory marker is a positive acute phase inflammatory marker.

19. The use of claim 18, wherein the positive acute phase inflammatory marker is c-reactive protein.

20. The use according to any one of claim 11 to 19, wherein the retrovirus is selected from the group consisting of HIV-1, HIV-2, HTLV-1 and HTLV-2.

21. The use according to any one of claims 11 to 20, wherein the mammalian subject is a human.

## Patentansprüche

1. Verfahren zur Bestimmung davon, wann ein Mittel, das die Produktion von T-Regulatorzellen hemmt, deren Funktion beschränkt und/oder diese Zellen zerstört, einem Säugerpatienten mit einer Retrovirusinfektion zu verabreichen ist, der einer Behandlung ausgesetzt wurde, durch die die Anzahl gegen das Retrovirus gerichteter CD8+-T-Effektor-zellen erhöht wird und/oder diese aktiviert werden, wobei man bei dem Verfahren eine zuvor dem Patienten nach der Behandlung entnommene Probe auf einen Akute-Phase-Entzündungsmarker analysiert, wobei Schwankungen bei den Spiegeln des Akute-Phase-Entzündungsmarkers als Hilfe bei der Bestimmung davon, wann das Mittel zu verabreichen ist, verwendet werden und wobei der Zeitpunkt der Verabreichung des Mittels so ausgewählt wird, dass es eine proportional größere Wirkung gegen die T-Regulatorzellen als die CD8+-T-Effektorzellen ausübt, und wobei das Mittel aus der aus antiproliferativen Arzneistoffen, Strahlung und einem Antikörper, der spezifisch an CD4+-T-Zellen bindet, bestehenden Gruppe ausgewählt ist;
wobei es sich bei dem Akute-Phase-Entzündungsmarker um einen positiven Akute-Phase-Entzündungsmarker handelt, der aus der folgenden Gruppe ausgewählt ist: c-reaktives Protein, Serumamyloid A, Serumamyloid-P-Komponente, Komplementproteine, ausgewählt aus C2-, C3-, C4-, C5-, C9-, B-, C1-Inhibitor und C4 bindendes Protein, Fibrinogen, Von-Willebrand-Faktor, α1-Antitrypsin, α1-Antichymotrypsin, α2-Antiplasmin, Heparin-Cofaktor II, Plasminogen-Aktivator-Inhibitor I, Haptoglobin, Hämopexin, Ceruloplasmin, Mangansuperoxid-Dismutase, α1-Säure-Glykoprotein, Hämoxygenase, Mannose bindendes Protein, Leukozytenprotein I, Lipoprotein (a) und Lipopolysaccharid bindendes Protein; oder
wobei es sich bei dem Akute-Phase-Entzündungsmarker um einen negativen Akute-Phase-Entzündungsmarker handelt, der aus der folgenden Gruppe ausgewählt ist: Albumin, Präalbumin, Transferin, apoAI, apoAII, α2-HS-Glykoprotein, Inter-α-Trypsin-Inhibitor und histidinreiches Glykoprotein 2.

2. Verfahren nach Anspruch 1, wobei es sich bei dem Akute-Phase-Entzündungsmarker um einen positiven Akute-Phase-Entzündungsmarker handelt.

3. Verfahren nach Anspruch 2, wobei es sich bei dem positiven Akute-Phase-Entzündungsmarker um c-reaktives Protein oder Serumamyloid A handelt.

4. Verfahren nach Anspruch 3, wobei es sich bei dem positiven Akute-Phase-Entzündungsmarker um c-reaktives Protein handelt.

5. Verfahren nach einem der Ansprüche 2 bis 4, wobei die Effektorzellenproduktion nach der Behandlung durch erhöhte Spiegel an positivem Akute-Phase-Entzündungsmarker angezeigt wird.

6. Verfahren nach Anspruch 1, wobei es sich bei dem Akute-Phase-Entzündungsmarker um einen negativen Akute-Phase-Entzündungsmarker handelt.

7. Verfahren nach Anspruch 6, wobei es sich bei dem negativen Akute-Phase-Entzündungsmarker um Albumin handelt.

8. Verfahren nach einem der Ansprüche 1 bis 7, wobei das Verfahren das Analysieren von dem Patienten wenigstens alle 48 Stunden entnommenen Proben umfasst.

9. Verfahren nach einem der Ansprüche 1 bis 7, wobei das Verfahren das Analysieren von dem Patienten wenigstens alle 24 Stunden entnommenen Proben umfasst.

10. Verfahren nach einem der Ansprüche 1 bis 10, wobei es sich bei der Behandlung, durch die die Anzahl gegen das Retrovirus gerichteter CD8+-T-Effektorzellen erhöht wird und/oder diese aktiviert werden, um (i) Absetzen der antiretroviralen Arzneistofftherapie oder (ii) eine Zusammensetzung, durch die die Anzahl gegen ein Retrovirus gerichteter Effektorzellen erhöht wird und/oder diese aktiviert werden, handelt und wobei die Zusammensetzung ein retrovirales antigenes Polypeptid oder für das Polypeptid codierende DNA umfasst.

11. Verwendung einer Zusammensetzung, durch die die Anzahl gegen ein Retrovirus gerichteter Effektorzellen erhöht wird und/oder diese aktiviert werden, zur Herstellung eines Arzneimittels zur Behandlung einer Retrovirusinfektion bei einem Säugerpatienten, wobei dem Patienten anschließend ein Mittel verabreicht wird, das die Produktion von T-Regulatorzellen hemmt, deren Funktion beschränkt und/oder diese Zellen zerstört, und wobei der Zeitpunkt der Verabreichung des Mittels so ausgewählt wird, dass es eine proportional größere Wirkung gegen die T-Regulatorzellen als die CD8+-T-Effektorzellen ausübt, und wobei Schwankungen bei den Spiegeln eines Akute-Phase-Entzündungsmarkers in dem Patienten als Hilfe bei der Bestimmung davon, wann das Mittel verabreicht wird, verwendet werden und wobei die Zusammensetzung ein retrovirales antigenes Polypeptid oder für das Polypeptid codierende DNA umfasst und wobei das Mittel aus der aus antiproliferativen Arzneistoffen, Strahlung und einem Antikörper, der spezifisch an CD4+-T-Zellen bindet, bestehenden Gruppe ausgewählt ist, wobei es sich bei dem Akute-Phase-Entzündungsmarker um einen positiven Akute-Phase-Entzündungsmarker handelt, der aus der folgenden Gruppe ausgewählt ist: c-reaktives Protein, Serumamyloid A, Serumamyloid-P-Komponente, Komplementproteine, ausgewählt aus C2-, C3-, C4-, C5-, C9-, B-, C1-Inhibitor und C4 bindendes Protein, Fibrinogen, Von-Willebrand-Faktor, α1-Antitrypsin, α1-Antichymotrypsin, α2-Antiplasmin, Heparin-Cofaktor II, Plasminogen-Aktivator-Inhibitor I, Haptoglobin, Hämopexin, Ceruloplasmin, Mangansuperoxid-Dismutase, α1-Säure-Glykoprotein, Hämoxygenase, Mannose bindendes Protein, Leukozytenprotein I, Lipoprotein (a) und Lipopolysaccharid bindendes Protein; oder
wobei es sich bei dem Akute-Phase-Entzündungsmarker um einen negativen Akute-Phase-Entzündungsmarker handelt, der aus der folgenden Gruppe ausgewählt ist: Albumin, Präalbumin, Transferin, apoAI, apoAII, α2-HS-Glykoprotein, Inter-α-Trypsin-Inhibitor und histidinreiches Glykoprotein.

12. Verwendung eines Mittels, das die Produktion von T-Regulatorzellen hemmt, deren Funktion beschränkt und/oder diese Zellen zerstört, zur Herstellung eines Arzneimittels zur Behandlung einer Retrovirusinfektion bei einem Säugerpatienten, wobei dem Patienten zuvor eine Zusammensetzung verabreicht wurde, durch die die Anzahl gegen ein Retrovirus gerichteter Effektorzellen erhöht wird und/oder diese aktiviert werden, und wobei das Mittel zu einem Zeitpunkt verabreicht wird, der so ausgewählt ist, dass es eine proportional größere Wirkung gegen die T-Regulatorzellen als die CD8+-T-Effektorzellen ausübt, und wobei Schwankungen bei den Spiegeln eines Akute-Phase-Entzündungsmarkers in dem Patienten als Hilfe bei der Bestimmung davon, wann das Mittel verabreicht wird, verwendet werden und wobei das Mittel aus der aus antiproliferativen Arzneistoffen, Strahlung und einem Antikörper, der spezifisch an CD4+-T-Zellen bindet, bestehenden Gruppe ausgewählt ist und wobei die Zusammensetzung ein retrovirales antigenes Polypeptid oder für das Polypeptid codierende DNA umfasst,
wobei es sich bei dem Akute-Phase-Entzündungsmarker um einen positiven Akute-Phase-Entzündungsmarker handelt, der aus der folgenden Gruppe ausgewählt ist: c-reaktives Protein, Serumamyloid A, Serumamyloid-P-Komponente, Komplementproteine, ausgewählt aus C2-, C3-, C4-, C5-, C9-, B-, C1-Inhibitor und C4 bindendes Protein, Fibrinogen, Von-Willebrand-Faktor, α1-Antitrypsin, α1-Antichymotrypsin, α2-Antiplasmin, Heparin-Cofaktor II, Plasminogen-Aktivator-Inhibitor I, Haptoglobin, Hämopexin, Ceruloplasmin, Mangansuperoxid-Dismutase, α1-Säure-Glykoprotein, Hämoxygenase, Mannose bindendes Protein, Leukozytenprotein I, Lipoprotein (a) und Lipopolysaccharid bindendes Protein; oder
wobei es sich bei dem Akute-Phase-Entzündungsmarker um einen negativen Akute-Phase-Entzündungsmarker handelt, der aus der folgenden Gruppe ausgewählt ist: Albumin, Präalbumin, Transferin, apoAI, apoAII, α2-HS-Glykoprotein, Inter-α-Trypsin-Inhibitor und histidirireiches Glykoprotein.

13. Verwendung nach Anspruch 11 oder Anspruch 12, wobei das Mittel ungefähr dann verabreicht wird, wenn die Anzahl der Viruspartikel nach Verabreichung der Zusammensetzung begonnnen hat, sich zu stabilisieren oder zu steigen, wobei mit dem Testen auf Viruspartikelspiegel in dem Patienten begonnen wird, wenn die Spiegel an positivem Akute-Phase-Entzündungsmarker nach Verabreichung der Zusammensetzung zu steigen beginnen.

14. Verwendung eines antiretroviralen Arzneistoffs zur Herstellung eines Arzneimittels zur Behandlung einer Retrovirusinfektion bei einem Säugerpatienten, wobei dem Patienten anschließend ein Mittel verabreicht wird, das die Produktion von T-Regulatorzellen hemmt, deren Funktion beschränkt und/oder diese Zellen zerstört, wobei das Mittel nach Beendigung der antiretroviralen Arzneistofftherapie und nachdem eine resultierende Expansion der Retrovirusanzahlen zu einer Erhöhung der Anzahl und/oder der Aktivierung von gegen das Retrovirus gerichteten Effektorzellen geführt hat verabreicht wird und wobei der Zeitpunkt der Verabreichung des Mittels so ausgewählt wird, dass es eine proportional größere Wirkung gegen die T-Regulatorzellen als die CD8+-T-Effektorzellen ausübt, und wobei Schwankungen bei den Spiegeln eines Akute-Phase-Entzündungsmarkers in dem Patienten als Hilfe bei der Bestimmung davon, wann das Mittel verabreicht wird, verwendet werden und wobei das Mittel aus der aus antiproliferativen Arzneistoffen, Strahlung und einem Antikörper, der spezifisch an CD4+-T-Zellen bindet, bestehenden Gruppe ausgewählt ist;
wobei es sich bei dem Akute-Phase-Entzündungsmarker um einen positiven Akute-Phase-Entzündungsmarker handelt, der aus der folgenden Gruppe ausgewählt ist: c-reaktives Protein, Serumamyloid A, Serumamyloid-P-Komponente, Komplementproteine, ausgewählt aus C2-, C3-, C4-, C5-, C9-, B-, C1-Inhibitor und C4 bindendes Protein, Fibrinogen, Von-Willebrand-Faktor, α1-Antitrypsin, α1-Anti-chymotrypsin, α2-Antiplasmin, Heparin-Cofaktor II, Plasminogen-Aktivator-Inhibitor I, Haptoglobin, Hämopexin, Ceruloplasmin, Mangansuperoxid-Dismutase, α1-Säure-Glykoprotein, Hämoxygenase, Mannose bindendes Protein, Leukozytenprotein I, Lipoprotein (a) und Lipopolysaccharid bindendes Protein; oder
wobei es sich bei dem Akute-Phase-Entzündungsmarker um einen negativen Akute-Phase-Entzündungsmarker handelt, der aus der folgenden Gruppe ausgewählt ist: Albumin, Präalbumin, Transferin, apoAI, apoAII, α2-HS-Glykoprotein, Inter-α-Trypsin-Inhibitor und histidinreiches Glykoprotein.

15. Verwendung eines Mittels, das die Produktion von T-Regulatorzellen hemmt, deren Funktion beschränkt und/oder diese Zellen zerstört, zur Herstellung eines Arzneimittels zur Behandlung einer Retrovirusinfektion bei einem Säugerpatienten, wobei dem Patienten zuvor ein antiretroviraler Arzneistoff verabreicht wurde, wobei das Mittel nach Beendigung der antiretroviralen Arzneistofftherapie und nachdem eine resultierende Expansion der Retrovirusanzahlen zu einer Erhöhung der Anzahl und/oder der Aktivierung von gegen das Retrovirus gerichteten Effektorzellen geführt hat verabreicht wird und wobei das Mittel zu einem Zeitpunkt verabreicht wird, der so ausgewählt ist, dass es eine proportional größere Wirkung gegen die T-Regulatorzellen als die CD8+-T-Effektorzellen ausübt, und wobei Schwankungen bei den Spiegeln eines Akute-Phase-Entzündungsmarkers in dem Patienten als Hilfe bei der Bestimmung davon, wann das Mittel verabreicht wird, verwendet werden und wobei das Mittel aus der aus antiproliferativen Arzneistoffen, Strahlung und einem Antikörper, der spezifisch an CD4+-T-Zellen bindet, bestehenden Gruppe ausgewählt ist,
wobei es sich bei dem Akute-Phase-Entzündungsmarker um einen positiven Akute-Phase-Entzündungsmarker handelt, der aus der folgenden Gruppe ausgewählt ist: c-reaktives Protein, Serumamyloid A, Serumamyloid-P-Komponente, Komplementproteine, ausgewählt aus C2-, C3-, C4-, C5-, C9-, B-, C1-Inhibitor und C4 bindendes Protein, Fibrinogen, Von-Willebrand-Faktor, α1-Antitrypsin, 1-Antichymotrypsin, α2-Antiplasmin, Heparin-Cofaktor II, Plasminogen-Aktivator-Inhibitor I, Haptoglobin, Hämopexin, Ceruloplasmin, Mangansuperoxid-Dismutase, α1-Säure-Glykoprotein, Hämoxygenase, Mannose bindendes Protein, Leukozytenprotein I, Lipoprotein (a) und Lipopolysaccharid bindendes Protein; oder
wobei es sich bei dem Akute-Phase-Entzündungsmarker um einen negativen Akute-Phase-Entzündungsmarker handelt, der aus der folgenden Gruppe ausgewählt ist: Albumin, Präalbumin, Transferin, apoAI, apoAII, α2-HS-Glykoprotein, Inter-α-Trypsin-Inhibitor und histidinreiches Glykoprotein.

16. Verwendung nach Anspruch 14 oder Anspruch 15, wobei das Mittel ungefähr dann verabreicht wird, wenn die Anzahl der Viruspartikel ihr Maximum erreicht hat oder nach Erreichen dieses Maximums zu sinken begonnnen hat, als Antwort auf die Beendigung der antiretroviralen Arzneistofftherapie, wobei mit dem Testen auf Viruspartikelspiegel in dem Patienten begonnen wird, wenn die Spiegel eines positiven Akute-Phase-Entzündungsmarkers nach Beendigung der antiretroviralen Arzneistofftherapie zu steigen beginnen.

17. Verwendung gemäß einem der Ansprüche 14 bis 16, wobei es sich bei der antiretroviralen Arzneistofftherapie um HAART handelt.

18. Verwendung gemäß einem der Ansprüche 11 bis 17, wobei es sich bei dem Akute-Phase-Entzündungsmarker um einen positiven Akute-Phase-Entzündungsmarker handelt.

19. Verwendung nach Anspruch 18, wobei es sich bei dem positiven Akute-Phase-Entzündungsmarker um c-reaktives Protein handelt.

20. Verwendung gemäß einem der Ansprüche 11 bis 19, wobei das Retrovirus aus der aus HIV-1, HIV-2, HTLV-1 und HTLV-2 bestehenden Gruppe ausgewählt ist.

21. Verwendung gemäß einem der Ansprüche 11 bis 20, wobei es sich bei dem Säugerpatienten um einen Menschen handelt.

## Revendications

1. Procédé pour déterminer à quel moment un agent qui inhibe la production de, limite la fonction de et/ou détruit, les lymphocytes T régulateurs, doit être administré à un sujet mammifère ayant une infection rétrovirale qui a été exposé à un traitement qui augmente le nombre de, et/ou active, les lymphocytes T CD8+ effecteurs dirigés contre le rétrovirus, le procédé comprenant l'analyse d'un échantillon précédemment obtenu à partir du sujet après le traitement pour un marqueur inflammatoire de phase aiguë, les fluctuations des taux d'un marqueur inflammatoire de phase aiguë chez le sujet étant utilisées pour aider à déterminer le moment auquel l'agent doit être administré, et le temps d'administration de l'agent étant choisi de sorte qu'il exerce un effet proportionnellement plus élevé contre les lymphocytes T régulateurs que les lymphocytes T CD8+ effecteurs, et l'agent étant choisi dans le groupe constitué de médicaments antiprolifératifs, un rayonnement et un anticorps qui se lie spécifiquement aux lymphocytes T CD4+ ;
le marqueur inflammatoire de phase aiguë étant un marqueur inflammatoire de phase aiguë positif choisi dans le groupe constitué de : la protéine C-réactive, l'amyloïde sérique A, le composant amyloïde sérique P, des protéines du complément choisies parmi C2, C3, C4, C5, C9, B, l'inhibiteur C1 et la protéine de liaison C4, le fibrinogène, le facteur de von Willebrand, l'α1-antitrypsine, l'α1-antichymotrypsine, l'α2-antiplasmine, le cofacteur d'héparine II, l'inhibiteur d'activateur du plasminogène I, l'haptoglobine, l'hémopexine, la céruloplasmine, la superoxyde de manganèse dismutase, l'α1-glycoprotéine acide, l'hème oxygénase, la protéine de liaison au mannose, la protéine leucocytaire I, la lipoprotéine (a) et la protéine de liaison au lipopolysaccharide ; ou le marqueur inflammatoire de phase aiguë étant un marqueur inflammatoire de phase aiguë négatif choisi dans le groupe constitué de : l'albumine, la pré-albumine, la transferrine, apoAI, apoAII, l'α2-HS-glycoprotéine, l'inhibiteur d'inter-α-trypsine et la glycoprotéine 2 riche en histidine.

2. Procédé de la revendication 1, le marqueur inflammatoire de phase aiguë étant un marqueur inflammatoire de phase aiguë positif.

3. Procédé de la revendication 2, le marqueur inflammatoire de phase aiguë positif étant la protéine C-réactive ou l'amyloïde sérique A.

4. Procédé de la revendication 3, le marqueur inflammatoire de phase aiguë positif étant la protéine C-réactive.

5. Procédé selon l'une quelconque des revendications 2 à 4, la production de cellules effectrices après le traitement étant indiquée par des taux augmentés de marqueur inflammatoire de phase aiguë positif.

6. Procédé de la revendication 1, le marqueur inflammatoire de phase aiguë étant un marqueur inflammatoire de phase aiguë négatif.

7. Procédé de la revendication 6, le marqueur inflammatoire de phase aiguë négatif étant l'albumine.

8. Procédé selon l'une quelconque des revendications 1 à 7, le procédé comprenant l'analyse d'échantillons obtenus au moins toutes les 48 heures à partir du sujet.

9. Procédé selon l'une quelconque des revendications 1 à 7, le procédé comprenant l'analyse d'échantillons obtenus au moins toutes les 24 heures à partir du sujet.

10. Procédé selon l'une quelconque des revendications 1 à 10, le traitement qui augmente le nombre de, et/ou active, les lymphocytes T CD8+ effecteurs dirigés contre le rétrovirus étant (i) le sevrage de la thérapie par médicament antirétroviral ou (ii) une composition qui augmente le nombre de et/ou active les cellules effectrices dirigées contre un rétrovirus, et la composition comprenant un polypeptide antigénique rétroviral ou un ADN codant pour le polypeptide.

11. Utilisation d'une composition qui augmente le nombre de et/ou active les cellules effectrices dirigées contre un rétrovirus pour la fabrication d'un médicament pour traiter une infection rétrovirale chez un sujet mammifère, le sujet recevant ensuite l'administration d'un agent qui inhibe la production de, limite la fonction de et/ou détruit, les lymphocytes T régulateurs, et le temps d'administration de l'agent étant choisi de sorte qu'il exerce un effet proportionnellement plus élevé contre les lymphocytes T régulateurs que les lymphocytes T CD8+ effecteurs, et les fluctuations des taux d'un marqueur inflammatoire de phase aiguë chez le sujet étant utilisées pour aider à déterminer le moment auquel l'agent est administré, et la composition comprenant un polypeptide antigénique rétroviral ou un ADN codant pour le polypeptide, et l'agent étant choisi dans le groupe constitué de médicaments antiprolifératifs, un rayonnement et un anticorps qui se lie spécifiquement aux lymphocytes T CD4+, le marqueur inflammatoire de phase aiguë étant un marqueur inflammatoire de phase aiguë positif choisi dans le groupe constitué de : la protéine C-réactive, l'amyloïde sérique A, le composant amyloïde sérique P, des protéines du complément choisies parmi C2, C3, C4, C5, C9, B, l'inhibiteur C1 et la protéine de liaison C4, le fibrinogène, le facteur de von Willebrand, l'α1-antitrypsine, l'α1-antichymotrypsine, l'α2-antiplasmine, le cofacteur d'héparine II, l'inhibiteur d'activateur du plasminogène I, l'haptoglobine, l'hémopexine, la céruloplasmine, la superoxyde de manganèse dismutase, l'α1-glycoprotéine acide, l'hème oxygénase, la protéine de liaison au mannose, la protéine leucocytaire I, la lipoprotéine (a) et la protéine de liaison au lipopolysaccharide ; ou
le marqueur inflammatoire de phase aiguë étant un marqueur inflammatoire de phase aiguë négatif choisi dans le groupe constitué de : l'albumine, la préalbumine, la transferrine, apoAI, apoAII, l'α2-HS-glycoprotéine, l'inhibiteur d'inter-α-trypsine et la glycoprotéine riche en histidine.

12. Utilisation d'un agent qui inhibe la production de, limite la fonction de et/ou détruit, les lymphocytes T régulateurs pour la fabrication d'un médicament pour traiter une infection rétrovirale chez un sujet mammifère, le sujet ayant précédemment reçu l'administration d'une composition qui augmente le nombre de et/ou active les cellules effectrices dirigées contre un rétrovirus, et l'agent étant administré à un temps choisi de sorte qu'il exerce un effet proportionnellement plus élevé contre les lymphocytes T régulateurs que les lymphocytes T CD8+ effecteurs, et les fluctuations des taux d'un marqueur inflammatoire de phase aiguë chez le sujet étant utilisées pour aider à déterminer le moment auquel l'agent est administré, et l'agent étant choisi dans le groupe constitué de médicaments antiprolifératifs, un rayonnement et un anticorps qui se lie spécifiquement aux lymphocytes T CD4+, et la composition comprenant un polypeptide antigénique rétroviral ou un ADN codant pour le polypeptide ;
le marqueur inflammatoire de phase aiguë étant un marqueur inflammatoire de phase aiguë positif choisi dans le groupe constitué de : la protéine C-réactive, l'amyloïde sérique A, le composant amyloïde sérique P, des protéines du complément choisies parmi C2, C3, C4, C5, C9, B, l'inhibiteur C1 et la protéine de liaison C4, le fibrinogène, le facteur de von Willebrand, l'α1-antitrypsine, l'α1-antichymotrypsine, l'α2-antiplasmine, le cofacteur d'héparine II, l'inhibiteur d'activateur du plasminogène I, l'haptoglobine, l'hémopexine, la céruloplasmine, la superoxyde de manganèse dismutase, l'α1-glycoprotéine acide, l'hème oxygénase, la protéine de liaison au mannose, la protéine leucocytaire I, la lipoprotéine (a) et la protéine de liaison au lipopolysaccharide ; ou le marqueur inflammatoire de phase aiguë étant un marqueur inflammatoire de phase aiguë négatif choisi dans le groupe constitué de : l'albumine, la pré-albumine, la transferrine, apoAI, apoAII, l'α2-HS-glycoprotéine, l'inhibiteur d'inter-α-trypsine et la glycoprotéine riche en histidine.

13. Utilisation de la revendication 11 ou la revendication 12, l'agent étant administré approximativement lorsque le nombre de particules virales a commencé à se stabiliser ou augmenter après l'administration de la composition, les tests du taux de particules virales chez le sujet commençant lorsque les taux de marqueur inflammatoire de phase aiguë positif commencent à augmenter après l'administration de la composition.

14. Utilisation d'un médicament antirétroviral pour la fabrication d'un médicament pour traiter une infection rétrovirale chez un sujet mammifère, le sujet recevant ensuite l'administration d'un agent qui inhibe la production de, limite la fonction de et/ou détruit, les lymphocytes T régulateurs, l'agent étant administré après que la thérapie par médicament antirétroviral soit terminée et qu'une expansion résultante des nombres rétroviraux ait conduit à une augmentation du nombre, et/ou l'activation, de cellules effectrices dirigées contre le rétrovirus, et le temps d'administration de l'agent étant choisi de sorte qu'il exerce un effet proportionnellement plus élevé contre les lymphocytes T régulateurs que les lymphocytes T CD8+ effecteurs, et les fluctuations des taux d'un marqueur inflammatoire de phase aiguë chez le sujet étant utilisées pour aider à déterminer le moment auquel l'agent est administré, et l'agent étant choisi dans le groupe constitué de médicaments antiprolifératifs, un rayonnement et un anticorps qui se lie spécifiquement aux lymphocytes T CD4+ ;
le marqueur inflammatoire de phase aiguë étant un marqueur inflammatoire de phase aiguë positif choisi dans le groupe constitué de : la protéine C-réactive, l'amyloïde sérique A, le composant amyloïde sérique P, des protéines du complément choisies parmi C2, C3, C4, C5, C9, B, l'inhibiteur C1 et la protéine de liaison C4, le fibrinogène, le facteur de von Willebrand, l'α1-antitrypsine, l'α1-antichymotrypsine, l'α2-antiplasmine, le cofacteur d'héparine II, l'inhibiteur d'activateur du plasminogène I, l'haptoglobine, l'hémopexine, la céruloplasmine, la superoxyde de manganèse dismutase, l'α1-glycoprotéine acide, l'hème oxygénase, la protéine de liaison au mannose, la protéine leucocytaire I, la lipoprotéine (a) et la protéine de liaison au lipopolysaccharide ; ou le marqueur inflammatoire de phase aiguë étant un marqueur inflammatoire de phase aiguë négatif choisi dans le groupe constitué de : l'albumine, la pré-albumine, la transferrine, apoAI, apoAII, l'α2-HS-glycoprotéine, l'inhibiteur d'inter-α-trypsine et la glycoprotéine riche en histidine.

15. Utilisation d'un agent qui inhibe la production de, limite la fonction de et/ou détruit, les lymphocytes T régulateurs pour la fabrication d'un médicament pour traiter une infection rétrovirale chez un sujet mammifère, le sujet ayant précédemment reçu l'administration d'un médicament antirétroviral, l'agent étant administré après que la thérapie par médicament antirétroviral soit terminée et qu'une expansion résultante des nombres rétroviraux ait conduit à une augmentation du nombre, et/ou l'activation, de cellules effectrices dirigées contre le rétrovirus, et l'agent étant administré à un temps choisi de sorte qu'il exerce un effet proportionnellement plus élevé contre les lymphocytes T régulateurs que les lymphocytes T CD8+ effecteurs, et les fluctuations des taux d'un marqueur inflammatoire de phase aiguë chez le sujet sont utilisées pour aider à déterminer le moment auquel l'agent est administré, et l'agent étant choisi dans le groupe constitué de médicaments antiprolifératifs, un rayonnement et un anticorps qui se lie spécifiquement aux lymphocytes T CD4+ ;
le marqueur inflammatoire de phase aiguë étant un marqueur inflammatoire de phase aiguë positif choisi dans le groupe constitué de : la protéine C-réactive, l'amyloïde sérique A, le composant amyloïde sérique P, des protéines du complément choisies parmi C2, C3, C4, C5, C9, B, l'inhibiteur C1 et la protéine de liaison C4, le fibrinogène, le facteur de von Willebrand, l'α1-antitrypsine, l'α1-antichymotrypsine, l'α2-antiplasmine, le cofacteur d'héparine II, l'inhibiteur d'activateur du plasminogène I, l'haptoglobine, l'hémopexine, la céruloplasmine, la superoxyde de manganèse dismutase, l'α1-glycoprotéine acide, l'hème oxygénase, la protéine de liaison au mannose, la protéine leucocytaire I, la lipoprotéine (a) et la protéine de liaison au lipopolysaccharide ; ou le marqueur inflammatoire de phase aiguë étant un marqueur inflammatoire de phase aiguë négatif choisi dans le groupe constitué de : l'albumine, la pré-albumine, la transferrine, apoAI, apoAII, l'α2-HS-glycoprotéine, l'inhibiteur d'inter-α-trypsine et la glycoprotéine riche en histidine.

16. Utilisation de la revendication 14 ou la revendication 15, l'agent étant administré approximativement lorsque le nombre de particules virales a atteint un pic ou commencé à diminuer après ce pic, en réponse à la conclusion de la thérapie par médicament antirétroviral, les tests des taux de particules virales chez le sujet commençant lorsque les taux d'un marqueur inflammatoire de phase aiguë positif commencent à augmenter après l'arrêt de la thérapie par médicament antirétroviral.

17. Utilisation selon l'une quelconque des revendications 14 à 16, la thérapie par médicament antirétroviral étant HAART.

18. Utilisation selon l'une quelconque des revendications 11 à 17, le marqueur inflammatoire de phase aiguë étant un marqueur inflammatoire de phase aiguë positif.

19. Utilisation de la revendication 18, le marqueur inflammatoire de phase aiguë positif étant la protéine C-réactive.

20. Utilisation selon l'une quelconque des revendications 11 à 19, le rétrovirus étant choisi dans le groupe constitué de VIH-1, VIH-2, HTLV-1 et HTLV-2.

21. Utilisation selon l'une quelconque des revendications 11 à 20, le sujet mammifère étant un humain.
